# EUROPEAN PATENT APPLICATION

(11) **EP 4 403 045 A2**
(43) Date of publication of application: **24.07.2024**
(21) Application number: 24175583.4
(22) Date of filing: 27.06.2022
(51) Int. Cl.: A23L 33/16

(54) **METHODS FOR MAKING HIGH INTENSITY SWEETENERS**

(30) Priority: 29.06.2021 US 202163216008 P; 31.08.2021 EP 21193962
(62) Divisional of application: 22744628.3
(71) Applicant: Firmenich Incorporated, Plainsboro, NJ 08536 (US)
(72) Inventor: NORIEGA, Chris Edano, Plainsboro, NJ 08536 (US); MANAM, Rama Rao, Plainsboro, NJ 08536 (US); PATRON, Andrew, Plainsboro, NJ 08536 (US)
(74) Representative: Strych, Sebastian

(57) **Abstract**

Provided herein include methods of making mogroside compounds, e.g., siamenoside I 1, compositions (for example host cells) for making the mogroside compounds, and the mogroside compounds made by the methods disclosed herein, and compositions (for example, cell lysates) and recombinant cells comprising the mogroside compounds (e.g., siamenoside I). Also provided herein are novel cucurbitadienol synthases and the use thereof.

## Description

### Field

The present disclosure relates to methods, systems and compositions for producing sweet tasting compounds, as well as compositions comprising the sweet tasting compounds.

### Background Description

The taste system provides sensory information about the chemical composition of the external world. Taste transduction is one of the most sophisticated forms of chemical-triggered sensation in animals. Signaling of taste is found throughout the animal kingdom, from simple metazoans to the most complex of vertebrates. Mammals are believed to have five basic taste modalities: sweet, bitter, sour, salty, and umami (the taste of monosodium glutamate, a.k.a. savory taste).

For centuries, various natural and unnatural compositions and/or compounds have been added to ingestible compositions, including foods and beverages, and/or orally administered medicinal compositions to improve their taste. Although it has long been known that there are only a few basic types of "tastes," the biological and biochemical basis of taste perception was poorly understood, and most taste improving or taste modifying agents have been discovered largely by simple trial and error processes.

With respect to the sweet taste, diabetes, and cardiovascular disease are health concerns on the rise globally, but are growing at alarming rates in the United States. Sugar and calories are key components that can be limited to render a positive nutritional effect on health. High-intensity sweeteners can provide the sweetness of sugar, with various taste qualities. Because they are many times sweeter than sugar, much less of the sweetener is required to replace the sugar.

High-intensity sweeteners have a wide range of chemically distinct structures and hence possess varying properties, such as, without limitation, odor, flavor, mouthfeel, and aftertaste. These properties, particularly flavor and aftertaste, are well known to vary over the time of tasting, such that each temporal profile is sweetener-specific.

There has been significant recent progress in identifying useful natural flavoring agents, such as for example sweeteners such as sucrose, fructose, glucose, erythritol, isomalt, lactitol, mannitol, sorbitol, xylitol, certain known natural terpenoids, flavonoids, or protein sweeteners. See, *e.g,* Kinghom, et al., "Noncariogenic Intense Natural Sweeteners," Med. Res. Rev. 18 (5) 347-360 (1998) (discussing discovered natural materials that are much more intensely sweet than common natural sweeteners such as sucrose, fructose, and the like.) Similarly, there has been recent progress in identifying and commercializing new artificial sweeteners, such as aspartame, saccharin, acesulfame-K, cyclamate, sucralose, and the like. See, *e.g.,* Ager, et al., Angew. Chem. Int. Ed. 37, 1802-1817 (1998). The entire contents of the references identified above are hereby incorporated herein by reference in their entirety.

Sweeteners such as saccharin and 6-methyl-1,2,3-oxathiazin-4(3H)-one-2,2-dioxide potassium salt (acesulfame potassium) are commonly characterized as having bitter and/or metallic aftertastes. Products prepared with 2,4-dihydroxybenzoic acid are claimed to display reduced undesirable aftertastes associated with sweeteners, and do so at concentrations below those concentrations at which their own tastes are perceptible. Also, high intensity sweeteners such as sucralose and aspartame are reported to have sweetness delivery problems, i.e., delayed onset and lingering of sweetness. See S. G. Wiet, et al., J. Food Sci., 58(3):599-602, 666 (1993).

There is a need for new sweetening compounds, sweet taste enhancers, and compositions containing such compounds and enhancers, having improved taste and delivery characteristics. In addition, there is a need for foods containing new sweetening compounds and/or sweet taste enhancers with such desirable characteristics.

Mogrosides isolated from the fruits of *Siraitia grosvenorii* are one of the most potent triterpene glycoside sweeteners known. Also known as Luo Han Guo, or monk fruit, the extract from *S. grosvenorii* fruits have been historically used in China for medicinal purposes and as a herbal sweetener. The sweet potency of these higher glycosylated mogrosides make them great candidates as alternative sweeteners. In this invention, we describe enzymatic routes for production of a potent mogroside, Siamenoside I.

### SUMMARY

Provided herein include a method of producing siamenoside I having the structure of formula (I): 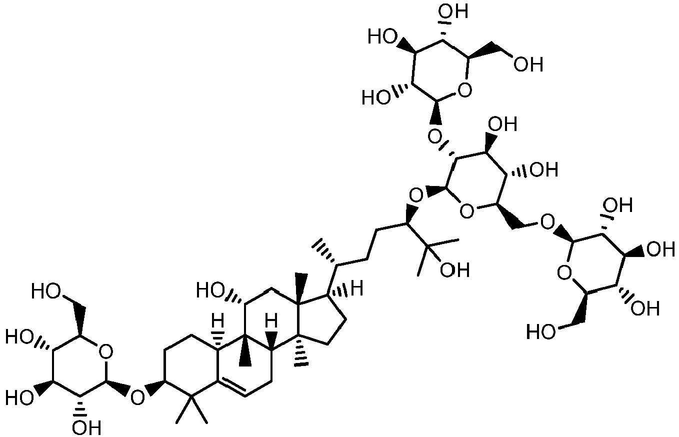 the method comprises contacting a mogroside having a structure of formula (II) 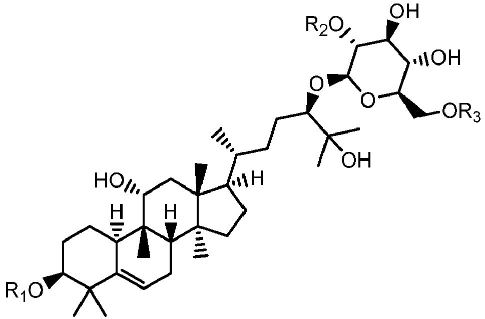 wherein R1, R2 and R3 is = glucose or H
with a polypeptide having UDP-glycotransferase activity.

In some embodiments, contacting a mogroside having a structure of formula (II) with a polypeptide having UDP-glycotransferase activity comprises contacting said mogroside having a structure of formula (II) with a recombinant host cell that comprises a gene encoding the polypeptide having UDP-glycotransferase activity.

In some embodiments, the mogroside having a structure of formula (II) contacts with the polypeptide having UDP-glycotransferase activity in a recombinant host cell that comprises a first polynucleotide encoding the polypeptide having UDP-glycotransferase activity.

The mogroside having a structure of formula (II) can be, for example, provided to the recombinant cell, present in the recombinant host cell, produced by the recombinant host cell, or any combination thereof. In some embodiments, the method comprises cultivating the recombinant host cell in a culture medium under conditions in which the polypeptide having UDP-glycotransferase activity is expressed.

In some embodiments, the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21 or SEQ ID NO: 23.

In some embodiments, the mogroside is Mog III and has a structure of formula (III)

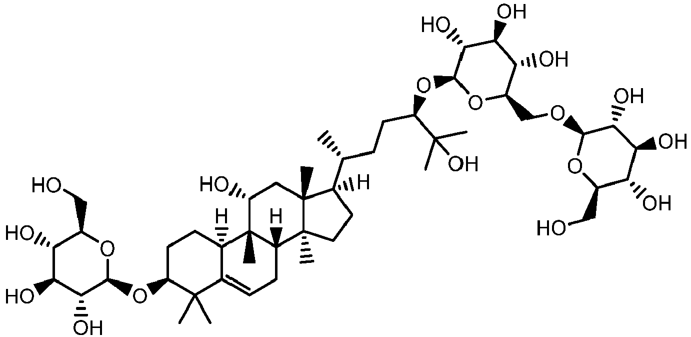

In some embodiments, where the method requires contacting mogroside III, the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 1 or SEQ ID NO: 3.

In some embodiments the mogroside is Mog IIIA and has a structure of formula (IV)

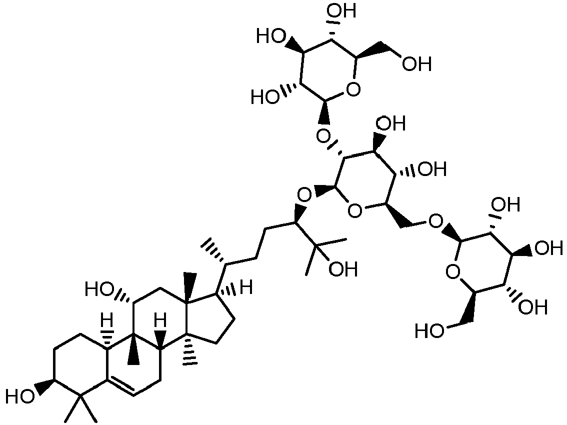

In some embodiments, where method requires contacting mogroside IIIA the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 5, SEQ ID NO: 7, or SEQ ID NO: 9, SEQ ID NO: 11, SEQ ID NO: 13, SEQ ID NO: 15, SEQ ID NO: 17 or SEQ ID NO: 19.

In some embodiments the mogroside is is Mog IIIE and has a structure of formula (V)

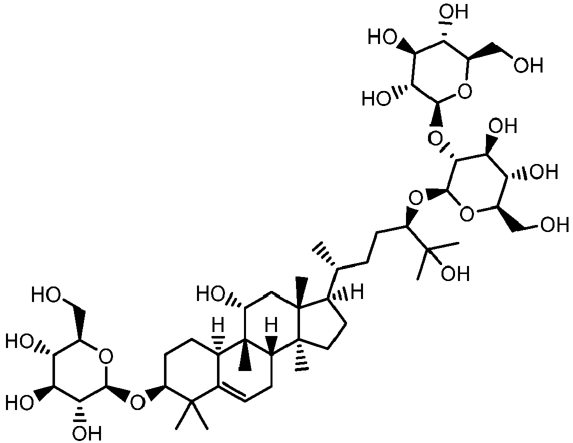

In some embodiments, where the method requires contacting mogroside IIIE the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following:, SEQ ID NO: 1, SEQ ID NO: 21 or SEQ ID NO: 23.

In a preferred embodiment where the method requires contacting mogroside IIIE, the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to SEQ ID NO: 21.

Also provided herein is a method of producing siamenoside I having the structure of formula (I): 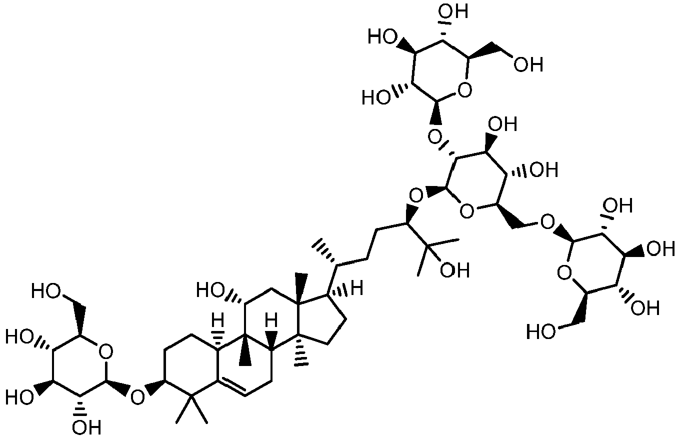 the method comprises contacting mogroside V having a structure of formula (VI) 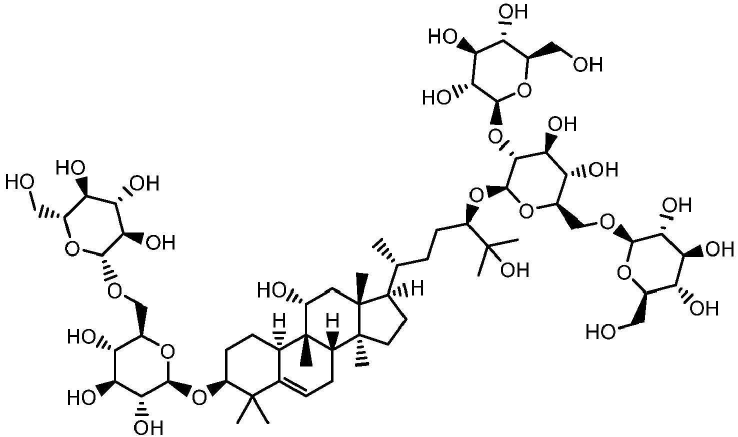 with a polypeptide having glucosidase activity.

In some embodiments, contacting a mogroside having a structure of formula (VI) with a polypeptide having glucosidase activity comprises contacting said mogroside having a structure of formula (VI) with a recombinant host cell that comprises a gene encoding the polypeptide having glucosidase activity.

In some embodiments, the mogroside having a structure of formula (VI) contacts with the polypeptide having glucosidase activity in a recombinant host cell that comprises a first polynucleotide encoding the polypeptide having glucosidase activity.

The mogroside having a structure of formula (VI) can be, for example, provided to the recombinant cell, present in the recombinant host cell, produced by the recombinant host cell, or any combination thereof. In some embodiments, the method comprises cultivating the recombinant host cell in a culture medium under conditions in which the polypeptide having glucosidase activity is expressed.

In a preferred embodiment where the method requires contacting mogroside having a structure of formula (VI), the polypeptide having glucosidase activity comprises an amino acid sequence having at least 40%, 45%, 50%, 55%, 60%, 65%, 70%, 75%, 80%, 85%, 90%, 95%, 96%, 97%, 98% or 99% sequence identity to SEQ ID NO: 25.

Provided herein include an expression vector comprising a nucleic acid molecule of nucleic acid encoding a UDP-glycotransferase activity, wherein the polypeptide comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21 or SEQ ID NO: 23.

Also provided herein include an expression vector comprising a nucleic acid molecule of nucleic acid encoding a glucosidase activity, wherein the polypeptide comprises an amino acid sequence having at least 40%, 45%, 50%, 55%, 60%, 65%, 70%, 75%, 80%, 85%, 90%, 95%, 96%, 97%, 98% or 99% sequence identity to SEQ ID NO: 25.

In some embodiments, where the vector is a prokaryotic vector, viral vector or a eukaryotic vector.

Provided herein include a host cell or a non-human host organism comprising
(i) a nucleic acid molecule of nucleic acid encoding a UDP-glycotransferase activity, wherein the polypeptide comprises an amino acid sequence having at least 40%, 45%, 50%, 55%, 60%, 65%, 70%, 75%, 80%, 85%, 90%, 95%, 96%, 97%, 98% or 99% sequence identity to SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21 or SEQ ID NO: 23.
(ii) the vector of any one of the embodiments described herein.

Also provided herein include a host cell or a non-human host organism comprising
(i) a nucleic acid molecule of nucleic acid encoding a glucosidase activity, wherein the polypeptide comprises an amino acid sequence having at least 40%, 45%, 50%, 55%, 60%, 65%, 70%, 75%, 80%, 85%, 90%, 95%, 96%, 97%, 98% or 99% sequence identity to SEQ ID NO: 25.
(ii) the vector of any one of the embodiments described herein.

In some embodiments, one or more of the genes encoding a UDP-glycotransferase or glucosidase is operably linked to a heterologous promoter. In some embodiments, the heterologous promoter is a CMV, EF1a, SV40, PGK1, human beta actin, CAG, GAL1, GAL10, TEF1, GDS, ADH1, CaMV35S, Ubi, T7, T7lac, Sp6, araBAD, trp, lac, Ptac, pL promoter, or a combination thereof. In some embodiments, the promoter is an inducible, repressible, or constitutive promoter. In some embodiments, production of one or more of pyruvate, acetyl-CoA, citrate, and TCA cycle intermediates have been upregulated in the recombinant host cell. In some embodiments, cytosolic localization has been upregulated in the recombinant host cell. In some embodiments, one or more of the first, second third, fourth, fifth, sixth, seventh, eighth, ninth, and tenth gene comprises at least one sequence encoding a 2A self-cleaving peptide.

In some embodiments, the recombinant host cell is a plant, bivalve, fish, fungus, bacteria, or mammalian cell. In some embodiments, the plant is selected from the group consisting of Siraitia, Momordica, Gynostemma, Cucurbita, Cucumis, Arabidopsis, Artemisia, Stevia, Panax, Withania, Euphorbia, Medicago, Chlorophytum, Eleutherococcus, Aralia, Morus, Medicago, Betula, Astragalus, Jatropha, Camellia, Hypholoma, Aspergillus, Solanum, Huperzia, Pseudostellaria, Corchorus, Hedera, Marchantia, and Morus. In some embodiments, the fungus is selected from the group consisting of *Trichophyton, Sanghuangporus, Taiwanofungus, Moniliophthora, Marssonina, Diplodia, Lentinula, Xanthophyllomyces, Pochonia, Colletotrichum, Diaporthe, Histoplasma, Coccidioides, Histoplasma, Sanghuangporus, Aureobasidium, Pochonia, Penicillium, Sporothrix, Metarhizium, Aspergillus, Yarrowia,* and *Lipomyces.* In some embodiments, the fungus is *Aspergillus nidulans, Yarrowia lipolytica,* or *Rhodosporin toruloides.* In some embodiments, the recombinant host cell is a yeast cell. In some embodiments, the yeast is selected from the group consisting of Candida, Sacccharaomyces, Saccharomycotina, Taphrinomycotina, Schizosaccharomycetes, Komagataella, Basidiomycota, Agaricomycotina, Tremellomycetes, Pucciniomycotina, Aureobasidium, Coniochaeta, Rhodosporidium, and Microboryomycetes. In some embodiments, the bacteria is selected from the group consisting of Frankia, Actinobacteria, Streptomyces, and Enterococcus. In some embodiments, the recombinant host cell is a *Saccharomyces cerevisiae* cell or a *Yarrowia lipolytica* cell. In some embodiments, one or more of the first, second third, fourth, fifth, sixth, seventh, eighth, ninth, and tenth gene is a codon optimized gene for expression in a bacterial, mammalian, plant, fungal and/or insect cell. In some embodiments, one or more of the first, second third, fourth, fifth, sixth, seventh, eighth, ninth, and tenth gene comprises a functional mutation to increased activity of the encoded enzyme. In some embodiments, cultivating the recombinant host cell comprises monitoring the cultivating for pH, dissolved oxygen level, nitrogen level, or a combination thereof of the cultivating conditions.

In some embodiments, the method comprises isolating siamenoside I. In some embodiments, isolating siamenoside I comprises lysing the recombinant host cell, and/or isolating siamenoside I from the culture medium. In some embodiments, the method comprises purifying siamenoside I. In some embodiments, purifying siamenoside I comprises HPLC, solid phase extraction or a combination thereof. In some embodiments, the purifying comprises harvesting the recombinant host cells; saving the supernatant; and lysing the recombinant host cells. In some embodiments, the lysing comprises subjecting the cells to shear force or detergent washes thereby obtaining a lysate. The shear force can be, for example, from a sonication method, french pressurized cells, or beads. In some embodiments, the lysate is subjected to filtering and purification steps. In some embodiments, the lysate is filtered and purified by solid phase extraction.

Disclosed herein include a siamenoside I having the structure of formula (I) as defined herein, wherein the compound is produced by any of the methods disclosed herein.

Disclosed herein include a cell lysate comprising siamenoside I having the structure of formula (I) as defined herein.

Also disclosed herein include a recombinant cell comprising siamenoside I having the structure of formula (I) as defined herein and a gene encoding a polypeptide having UDP-glycotransferase activity. In some embodiments, the gene is a heterologous gene to the recombinant cell.

Also disclosed herein include a recombinant cell comprising siamenoside I having the structure of formula (I) as defined herein and a gene encoding a polypeptide having glucosidase activity. In some embodiments, the gene is a heterologous gene to the recombinant cell.

In some embodiments, the cell is a mammalian, plant, bacterial, fungal, or insect cell. For example, the cell can be a yeast cell. In some embodiments, the yeast is selected from Candida, Saccharaomyces, Saccharomycotina, Taphrinomycotina, Schizosaccharomycetes, Komagataella, Basidiomycota, Agaricomycotina, Tremellomycetes, Pucciniomycotina, Aureobasidium, Coniochaeta, and Microboryomycetes. In some embodiments, the plant is selected from the group consisting of Siraitia, Momordica, Gynostemma, Cucurbita, Cucumis, Arabidopsis, Artemisia, Stevia, Panax, Withania, Euphorbia, Medicago, Chlorophytum, Eleutherococcus, Aralia, Morus, Medicago, Betula, Astragalus, Jatropha, Camellia, Hypholoma, Aspergillus, Solanum, Huperzia, Pseudostellaria, Corchorus, Hedera, Marchantia, and Morus. In some embodiments, the fungus is selected from Trichophyton, Sanghuangporus, Taiwanofungus, Moniliophthora, Marssonina, Diplodia, Lentinula, Xanthophyllomyces, Pochonia, Colletotrichum, Diaporthe, Histoplasma, Coccidioides, Histoplasma, Sanghuangporus, Aureobasidium, Pochonia, Penicillium, Sporothrix, and Metarhizium.

Disclosed herein include a recombinant nucleic acid molecule which comprises a nucleic acid sequence encoding any of the polypeptides having UDP-glycotransferase or glucosidase activity disclosed herein. Disclosed herein include a recombinant cell comprising any of the polypeptides having UDP-glycotransferase activity or glucosidase disclosed herein and/or any recombinant nucleic acid molecules disclosed herein encoding a polypeptides having UDP-glycotransferase or glucosidase activity. Also disclosed herein include a method using any of the polypeptides having UDP-glycotransferase or glucosidase activity disclosed herein. The method can comprise contacting a substrate for UDP-glycotransferase activity or glucosidase with a polypeptide having UDP-glycotransferase or glucosidase activity activity. In some embodiments, the contacting results in a production of siameoside I, the substrate for UDP-glycotransferase activity or glucosidase comprises one or more mogrosides of formula (II) or one or more mogrosides of formula (VI). In some embodiments, the contacting comprises contacting the substrate with a recombinant host cell which comprises a nucleic acid sequence encoding the UDP-glycotransferase activity or glucosidase. The recombinant host cell can, for example, express the UDP-glycotransferase or glucosidase activity. In some embodiments, the substrate is provided to, present in, and/or produced by the recombinant host cell.

Also disclosed herein include the use of any of the compositions disclosed herein, for example the composition comprising siamnoside I and at least one additional sweetener and/or sweek modifier to convey, enhance, modify, or improve the perception of sweetness of a consumable product.

### BRIEF DESCRIPTION OF THE DRAWINGS

**Figure 1****.** Glycosylation of Mogroside IIIE (MIIIE) by SEQ ID NO:21 (UGT330) to produce Siamenoside I (SI). Mogroside IVE (MIVE) and Mogroside V (MV) were produced as byproducts.
**Figure 2****.** Production of Siamenoside I (SI) from mogroside extract using *S. cerevisiae* (Δ*exg1*) with overexpression of hydrolytic enzyme from *Brettanomyces bruxellensis* (SEQ ID NO: 25). Mogroside V (MV), 11-oxo-Siamenoside I (Sl-oxo), Mogroside IIIE (MIIIE).

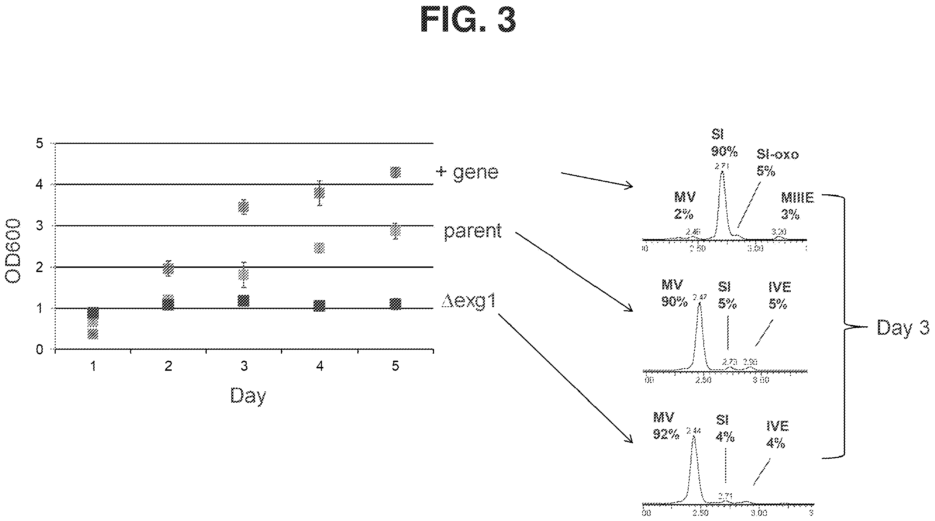

**Figure 3****.** Growth of different *S. cerevisiae* strains on mogroside extract *S. cerevisiae* (Δ*exg1*) with overexpression of SEQ ID NO: 25 (+ gene), parent (BY4741) and, *S. cerevisiae* (Δ*exg1*) only. Siamenoside I (SI), Mogroside V (MV), Mogroside IVE (IVE). Error bars are from biological replicates (n=3, mean and SE).

### DETAILED DESCRIPTION

### Definitions

Unless defined otherwise, all technical and scientific terms used herein have the same meaning as is commonly understood by one of ordinary skill in the art to which this disclosure belongs. All patents, applications, published applications, and other publications are incorporated by reference in their entirety. In the event that there is a plurality of definitions for a term herein, those in this section prevail unless stated otherwise.

"Solvate" refers to the compound formed by the interaction of a solvent and a compound described herein or salt thereof. Suitable solvates are physiologically acceptable solvates including hydrates.

A "sweetener", "sweet flavoring agent", "sweet flavor entity", "sweet compound," or "sweet tasting compound," as used herein refers to a compound or physiologically acceptable salt thereof that elicits a detectable sweet flavor in a subject. A "sweet modifier," as used herein refers to a compound or physiologically acceptable salt thereof that enhances, modifies, or improves the perception of sweetness.

As used herein, the term "operably linked" is used to describe the connection between regulatory elements and a gene or its coding region. Typically, gene expression is placed under the control of one or more regulatory elements, for example, without limitation, constitutive or inducible promoters, tissue-specific regulatory elements, and enhancers. A gene or coding region is said to be "operably linked to" or "operatively linked to" or "operably associated with" the regulatory elements, meaning that the gene or coding region is controlled or influenced by the regulatory element. For instance, a promoter is operably linked to a coding sequence if the promoter effects transcription or expression of the coding sequence.

The term "regulatory element" and "expression control element" are used interchangeably and refer to nucleic acid molecules that can influence the expression of an operably linked coding sequence in a particular host organism. These terms are used broadly to and cover all elements that promote or regulate transcription, including promoters, enhancer sequences, response elements, protein recognition sites, inducible elements, protein binding sequences, 5' and 3' untranslated regions (UTRs), transcriptional start sites, termination sequences, polyadenylation sequences, intrans, core elements required for basic interaction of RNA polymerase and transcription factors, upstream elements, enhancers, response elements (*see, e.g.,* Lewin, "Genes V" (Oxford University Press, Oxford) pages 847-873), and any combination thereof. Exemplary regulatory elements in prokaryotes include promoters, operator sequences and a ribosome binding sites. Regulatory elements that are used in eukaryotic cells can include, without limitation, transcriptional and translational control sequences, such as promoters, enhancers, splicing signals, polyadenylation signals, terminators, protein degradation signals, internal ribosome-entry element (IRES), 2A sequences, and the like, that provide for and/or regulate expression of a coding sequence and/or production of an encoded polypeptide in a host cell. In some embodiments herein, the recombinant cell described herein comprises a genes operably linked to regulatory elements.

As used herein, 2A sequences or elements refer to small peptides introduced as a linker between two proteins, allowing autonomous intraribosomal self-processing of polyproteins (*See e.g.,* de Felipe. Genetic Vaccines and Ther. 2:13 (2004); deFelipe et al. Traffic 5:616-626 (2004)). These short peptides allow co-expression of multiple proteins from a single vector. Many 2A elements are known in the art. Examples of 2A sequences that can be used in the methods and system disclosed herein, without limitation, include 2A sequences from the foot-and-mouth disease virus (F2A), equine rhinitis A virus (E2A), *Thosea asigna* virus (T2A), and porcine teschovirus-1 (P2A) as described in U.S. Patent Publication No. 20070116690.

As used herein, the term "promoter" is a nucleotide sequence that permits binding of RNA polymerase and directs the transcription of a gene. Typically, a promoter is located in the 5' non-coding region of a gene, proximal to the transcriptional start site of the gene. Sequence elements within promoters that function in the initiation of transcription are often characterized by consensus nucleotide sequences. Examples of promoters include, but are not limited to, promoters from bacteria, yeast, plants, viruses, and mammals (including humans). A promoter can be inducible, repressible, and/or constitutive. Inducible promoters initiate increased levels of transcription from DNA under their control in response to some change in culture conditions, such as a change in temperature.

As used herein, the term "enhancer" refers to a type of regulatory element that can increase the efficiency of transcription, regardless of the distance or orientation of the enhancer relative to the start site of transcription.

As used herein, the term "transgene" refers to any nucleotide or DNA sequence that is integrated into one or more chromosomes of a target cell by human intervention. In some embodiment, the transgene comprises a polynucleotide that encodes a protein of interest. The protein-encoding polynucleotide is generally operatively linked to other sequences that are useful for obtaining the desired expression of the gene of interest, such as transcriptional regulatory sequences. In some embodiments, the transgene can additionally comprise a nucleic acid or other molecule(s) that is used to mark the chromosome where it has integrated.

"Percent (%) sequence identity" with respect to polynucleotide or polypeptide sequences is used herein as the percentage of bases or amino acid residues in a candidate sequence that are identical with the bases or amino acid residues in another sequence, after aligning the two sequences. Gaps can be introduced into the sequence alignment, if necessary, to achieve the maximum percent sequence identity. Conservative substitutions are not considered as part of the sequence identity. Alignment for purposes of determining percent (%) sequence identity can be achieved in various ways that are within the skill in the art, for instance, using publicly available computer methods and programs such as BLAST, BLAST-2, ALIGN, FASTA (available in the Genetics Computing Group (GCG) package, from Madison, Wisconsin, USA), or Megalign (DNASTAR). Those of skill in the art can determine appropriate parameters for measuring alignment, including any algorithms needed to achieve maximal alignment over the full length of the sequences being compared.

For instance, percent (%) amino acid sequence identity values may be obtained by using the WU-BLAST-2 computer program described in, for example, Altschul et al., Methods in Enzymology, 1996, 266:460-480. Many search parameters in the WU-BLAST-2 computer program can be adjusted by those skilled in the art. For example, some of the adjustable parameters can be set with the following values: overlap span = 1, overlap fraction = 0.125, word threshold (T) = 11, and scoring matrix = BLOSUM62. When WU-BLAST-2 is used, a % amino acid sequence identity value is determined by dividing (a) the number of matching identical amino acid residues between the amino acid sequence of a first protein of interest and the amino acid sequence of a second protein of interest as determined by WU-BLAST-2 by (b) the total number of amino acid residues of the first protein of interest.

Percent amino acid sequence identity may also be determined using the sequence comparison program NCBI-BLAST2 described in, for example, Altschul et al., Nucleic Acids Res., 1997, 25:3389-3402. The NCBI-BLAST2 sequence comparison program may be downloaded from http://www.ncbi.nlm.nih.gov or otherwise obtained from the National Institute of Health, Bethesda, MD. NCBI-BLAST2 uses several adjustable search parameters. The default values for some of those adjustable search parameters are, for example, unmask = yes, strand = all, expected occurrences = 10, minimum low complexity length = 15/5, multi-pass e-value = 0.01, constant for multi-pass = 25, drop-off for final gapped alignment = 25 and scoring matrix = BLOSUM62.

In situations where NCBI-BLAST2 is used for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y, where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

As used herein, "isolated" means that the indicated compound has been separated from its natural milieu, such that one or more other compounds or biological agents present with the compound in its natural state are no longer present.

As used herein, "purified" means that the indicated compound is present at a higher amount relative to other compounds typically found with the indicated compound (e.g., in its natural environment). In some embodiments, the relative amount of purified a purified compound is increased by greater than 1%, 5%, 10%, 20%, 30%, 40%, 50%, 80%, 90%, 100%, 120%, 150%, 200%, 300%, 400%, or 1000%. In some embodiments, a purified compound is present at a weight percent level greater than 1%, 5%, 10%, 20%, 30%, 40%, 50%, 60%, 70%, 80%, 90%, 95%, 98%, 99%, or 99.5% relative to other compounds combined with the compound. In some embodiments, the siamenoside I produced from the embodiments herein is present at a weight percent level greater than 1%, 5%, 10%, 20%, 30%, 40%, 50%, 60%, 70%, 80%, 90%, 95%, 98%, 99%, or 99.5% relative to other compounds combined with the compound after production.

"Purification" as described herein, can refer to the methods for extracting siamenoside I from the cell lysate and/or the supernatant, wherein the cell is excreting the product of siamenoside I. "Lysate" as described herein, comprises the cellular content of a cell after disruption of the cell wall and cell membranes and can include proteins, sugars, and mogrosides, for example. Purification can involve ammonium sulfate precipitation to remove proteins, salting to remove proteins, hydrophobic separation (HPLC), and use of an affinity column. In view of the products produced by the methods herein, affinity media is contemplated for the removal of specific mogrosides with an adsorbent resin.

"HPLC" as described herein is a form of liquid chromatography that can be used to separate compounds that are dissolved in solution. Without being limiting the HPLC instruments can comprise of a reservoir of mobile phase, a pump, an injector, a separation column, and a detector. Compounds can then be separated by injecting a sample mixture onto the column. The different components in the mixture pass can pass through the column at different rates due to differences in their partitioning behavior between the mobile liquid phase and the stationary phase. There are several columns that can be used. Without being limiting the columns can be nomal phase columns, reverse phase columns, size exclusion type of columns, and ion exchange columns.

Also contemplated is the use of solid phase extraction and fractionation, which is useful for desalting proteins and sugar samples. Other methods can include the use of HPLC, liquid chromatography for analyzing samples, and liquid-liquid extraction, described in Auréa Andrade-Eiroa et al. (TrAC Trends in Analytical Chemistry Volume 80, June 2016, Pages 641-654; incorporated by reference in its entirety herein.

"Solid phase extraction" (SPE) for purification, as described herein, refers to a sample preparation process in which compounds that are dissolved or suspended in a liquid mixture are separated from other compounds in the mixture according to their physical and chemical properties. For example, analytical laboratories can use solid phase extraction to concentrate and purify samples for analysis. Solid phase extraction can also be used to isolate analytes of interest from a wide variety of matrices, including urine, blood, water, beverages, soil, and animal tissue, for example. In the embodiments herein, siameoside I that is in cell lysate or in the cell media can be purified by solid phase extraction.

SPE uses the affinity of solutes dissolved or suspended in a liquid (known as the mobile phase) for a solid through which the sample is passed (known as the stationary phase) to separate a mixture into desired and undesired components. SPE can also be used and applied directly in gas-solid phase and liquid-solid phase, or indirectly to solid samples by using, e.g., thermodesorption with subsequent chromatographic analysis. This can result in either the desired analytes of interest or undesired impurities in the sample are retained on the stationary phase. The portion that passes through the stationary phase can be collected or discarded, depending on whether it contains the desired analytes or undesired impurities. If the portion retained on the stationary phase includes the desired analytes, they can then be removed from the stationary phase for collection in an additional step, in which the stationary phase is rinsed with an appropriate eluent.

Ways that the solid phase extraction can be performed are not limited. Without being limiting, the procedures may include: Normal phase SPE procedure, Reversed phase SPE, Ion exchange SPE, Anion exchange SPE, Cation exchange, and Solid-phase microextraction. Solid phase extraction is described in Sajid *et al.,* and P 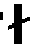 otka-Wasylka J *et al.* (Anal Chim Acta. 2017 May 1;965:36-53, Crit Rev Anal Chem. 2017 Apr 11:1-11; incorporated by reference in its entirety).

In some embodiments, the siameoside I that is produced by the cell is purified by solid phase extraction. In some embodiments, the purity of siameoside I, for example purified by solid phase extraction is 70%, 80%, 90% or 100% pure or any level of purity defined by any aforementioned values.

"Fermentation" as described herein, refers broadly to the bulk growth of host cells in a host medium to produce a specific product. In the embodiments herein, the final product produced is siameoside I. This can also include methods that occur with or without air and can be carried out in an anaerobic environment, for example. The whole cells (recombinant host cells) may be in fermentation broth or in a reaction buffer.

Siameoside I and intermediate mogroside compounds for the production of C siameoside I can be isolated by collection of intermediate mogroside compounds and siameoside I from the recombinant cell lysate or from the supernatant. The lysate can be obtained after harvesting the cells and subjecting the cells to lysis by shear force (French press cell or sonication) or by detergent treatment. The lysate can then be filtered and treated with ammonium sulfate to remove proteins, and fractionated on a C18 HPLC (5 X 10 cm Atlantis prep T3 OBD column, 5 um, Waters) and by injections using an A/B gradient (A = water B = acetonitrile) of 10 → 30% B over 30 minutes, with a 95% B wash, followed by re-equilibration at 1% (total run time = 42 minutes). The runs can be collected in tared tubes (12 fractions/plate, 3 plates per run) at 30 mL/fraction. The lysate can also be centrifuged to remove solids and particulate matter.

Purification of mogrosides can be performed using standard HPLC methods to attain purity levels of at least 95% using techniques well known to the skilled person.

As used herein, a "glycosidic bond" refers to a covalent bond connecting two furanose and/or pyranose groups together. Generally, a glycosidic bond is the bond between the anomeric carbon of one furanose or pyranose moiety and an oxygen of another furanose or pyranose moiety. Glycosidic bonds are named using the numbering of the connected carbon atoms, and the alpha/beta orientation. α- and β-glycosidic bonds are distinguished based on the relative stereochemistry of the anomeric position and the stereocenter furthest from C1 in the ring. For example, sucrose is a disaccharide composed of one molecule of glucose and one molecule of fructose connected through an alpha 1-2 glycosidic bond, as shown below.

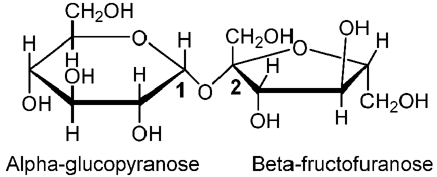

An example of a beta 1-4 glycosidic bond can be found in cellulose:

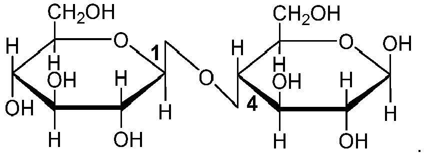

As used in the specification and the appended claims, the singular forms "a," "an" and "the" include plural referents unless the context clearly dictates otherwise. Thus, for example, reference to "an aromatic compound" includes mixtures of aromatic compounds.

Often, ranges are expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

"Codon optimization" as described herein, refers to the design process of altering codons to codons known to increase maximum protein expression efficiency. In some alternatives, codon optimization for expression in a cell is described, wherein codon optimization can be performed by using algorithms that are known to those skilled in the art so as to create synthetic genetic transcripts optimized for high mRNA and protein yield in humans. Codons can be optimized for protein expression in a bacterial cell, mammalian cell, yeast cell, insect cell, or plant cell, for example. Programs containing algorithms for codon optimization in humans are readily available. Such programs can include, for example, OptimumGene^{™} or GeneGPS^{®} algorithms. Additionally codon optimized sequences can be obtained commercially, for example, from Integrated DNA Technologies. In some of the embodiments herein, a recombinant cell for the production of siamenoside I comprises genes encoding enzymes for synthesis, wherein the genes are codon optimized for expression. In some embodiments, the genes are codon optimized for expression in bacterial, yeast, fungal or insect cells.

As used herein, the terms "nucleic acid," "nucleic acid molecule," and "polynucleotide" are interchangeable and refer to any nucleic acid, whether composed of phosphodiester linkages or modified linkages such as phosphotriester, phosphoramidate, siloxane, carbonate, carboxymethylester, acetamidate, carbamate, thioether, bridged phosphoramidate, bridged methylene phosphonate, bridged phosphoramidate, bridged phosphoramidate, bridged methylene phosphonate, phosphorothioate, methylphosphonate, phosphorodithioate, bridged phosphorothioate or sultone linkages, and combinations of such linkages. The terms "nucleic acid" and "polynucleotide" also specifically include nucleic acids composed of bases other than the five biologically occurring bases (adenine, guanine, thymine, cytosine and uracil).

Non-limiting examples of polynucleotides include deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid molecule" also includes so-called "peptide nucleic acids," which comprise naturally-occurring or modified nucleic acid bases attached to a polyamide backbone. Nucleic acids can be either single stranded or double stranded. In some alternatives, a nucleic acid sequence encoding a fusion protein is provided. In some alternatives, the nucleic acid is RNA or DNA. In some embodiments, the nucleic acid comprises any one of the relevant SEQ ID NOs: 1-26.

"Coding for" or "encoding" are used herein, and refers to the property of specific sequences of nucleotides in a polynucleotide, such as a gene, a cDNA, or an mRNA, to serve as templates for synthesis of other macromolecules such as a defined sequence of amino acids. Thus, a gene codes for a protein if transcription and translation of mRNA corresponding to that gene produces the protein in a cell or other biological system. In some embodiments herein, a recombinant cell is provided, wherein the recombinant cell comprises genes encoding for UDP glycosyltransferases or glucosidases. In some embodiments, the UDP glycosyltransferases comprises an amino acid sequence set forth by any one of SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21 or SEQ ID NO: 23. In some embodiments, the glucosidases comprises an amino acid sequence set forth by SEQ ID NO: 25. In some embodiments, the UDP glycosyltransferases are encoded by SEQ ID NO: 2, SEQ ID NO: 4, SEQ ID NO: 6, SEQ ID NO: 8, or SEQ ID NO: 10, SEQ ID NO: 12, SEQ ID NO: 14, SEQ ID NO: 16, SEQ ID NO: 18, SEQ ID NO: 20, SEQ ID NO: 22 or SEQ ID NO: 24.. In some embodiments, the glucosidases are encoded by SEQ ID NO: 26. In some embodiments, the genes encoding the UDP glycosyltransferases are codon optimized for expression in the host cell. A "nucleic acid sequence coding for a polypeptide" includes all nucleotide sequences that are degenerate versions of each other and that code for the same amino acid sequence.

Optimization can also be performed to reduce the occurrence of secondary structure in a polynucleotide. In some alternatives of the method, optimization of the sequences in the vector can also be performed to reduce the total GC/AT ratio. Strict codon optimization can lead to unwanted secondary structure or an undesirably high GC content that leads to secondary structure. As such, the secondary structures affect transcriptional efficiency. Programs such as GeneOptimizer can be used after codon usage optimization, for secondary structure avoidance and GC content optimization. These additional programs can be used for further optimization and troubleshooting after an initial codon optimization to limit secondary structures that can occur after the first round of optimization. Alternative programs for optimization are readily available. In some alternatives of the method, the vector comprises sequences that are optimized for secondary structure avoidance and/or the sequences are optimized to reduce the total GC/AT ratio and/or the sequences are optimized for expression in a bacterial or yeast cell.

"Vector," "Expression vector" or "construct" is a nucleic acid used to introduce heterologous nucleic acids into a cell that has regulatory elements to provide expression of the heterologous nucleic acids in the cell. Vectors include but are not limited to plasmid, minicircles, yeast, and viral genomes. In some alternatives, the vectors are plasmid, minicircles, yeast, or genomes. In some alternatives, the vector is for protein expression in a bacterial system such as *E*. *coli.* In some alternatives, the vector is for protein expression in a bacterial system, such as *E*. *coli.* In some alternatives, the vector is for protein expression in a yeast system. In some embodiments, the vector for expression is a viral vector. In some embodiments the vector is a recombinant vector comprising promoter sequences for upregulation of expression of the genes. "Regulatory elements" can refer to the nucleic acid that has nucleotide sequences that can influence the transcription or translation initiation and rate, stability and mobility of a transcription or translation product.

"Recombinant host" or "recombinant host cell" as described herein is a host, the genome of which has been augmented by at least one incorporated DNA sequence. Said incorporated DNA sequence may be a heterologous nucleic acid encoding one or more polypeptides. Such DNA sequences include but are not limited to genes that are not naturally present, DNA sequences that are not normally transcribed into RNA or translated into a protein ("expressed"), and other genes or DNA sequences which one desires to introduce into the nonrecombinant host. In some embodiments, the recombinant host cell is used to prevent expression problems such as codon-bias. There are commercial hosts for expression of proteins, for example, BL21-CodonPlus^{™} cells, tRNA-Supplemented Host Strains for Expression of Heterologous Genes, Rosetta^{™} (DE3) competent strains for enhancing expression of proteins, and commercial yeast expression systems in the genera *Saccharomyces, Pichia, Kluyveromyces, Hansenula* and *Yarrowia.*

The recombinant host may be a commercially available cell such as Rosetta cells for expression of enzymes that may have rare codons.

The type of host cell can vary. For example, the host cell can be selected from a group consisting of *Agaricus, Aspergillus, Bacillus, Candida, corynebacterium, Escherichia, Fusarium*/*Gibberella, Kluyveromyces, Laetiporus, Lentinus, Phaffia, Phanerochaete, Pichia, Physcomitrella, Rhodoturula, Saccharomyces, Schizosaccharomyces, Sphaceloma, Xanthophyllomyces, Yarrowia, Lentinus tigrinus, Laetiporus sulphureus, Phanerochaete chrysosporium, Pichia pastoris, Physcomitrella patens, Rhodoturula glutinis, Rhodoturula mucilaginosa, Phaffia rhodozyma, Xanthophyllomyces dendrorhous, Fusarium fujikuroi*/*Gibberella fujikuroi, Candida utilis, Yarrowia lipolytica, Siraitia, Momordica, Gynostemma, Cucurbita, Cucumis, Arabidopsis, Artemisia, Stevia, Panax, Withania, Euphorbia, Medicago, Chlorophytum, Eleutherococcus, Aralia, Morus, Medicago, Betula, Astragalus, Jatropha, Camellia, Hypholoma, Aspergillus, Solanum, Huperzia, Pseudostellaria, Corchorus, Hedera, Marchantia, and Morus, Trichophyton, Sanghuangporus, Taiwanofungus, Moniliophthora, Marssonina, Diplodia, Lentinula, Xanthophyllomyces, Pochonia, Colletotrichum, Diaporthe, Histoplasma, Coccidioides, Histoplasma, Sanghuangporus, Aureobasidium, Pochonia, Penicillium, Sporothrix, Metarhizium, Aspergillus, Yarrowia, Lipomyces, Aspergillus nidulans, Yarrowia lipolytica, Rhodosporin toruloides, Candida, Sacccharaomyces, Saccharomycotina, Taphrinomycotina, Schizosaccharomycetes, Komagataella, Basidiomycota, Agaricomycotina, Tremellomycetes, Pucciniomycotina, Aureobasidium, Coniochaeta, Rhodosporidium,* and *Microboryomycetes, Gibberella fujikuroi, Kluyveromyces lactis, Schizosaccharomyces pombe, Aspergillus niger, Saccharomyces cerevisiae, Escherichia coli, Rhodobacter sphaeroides,* and *Rhodobacter capsulatus.* Methods to enhance product yield have been described, for example, in *S. cerevisiae.* Methods are known for making recombinant microorganisms.

Methods to prepare recombinant host cells from *Aspergillus spp.* is described in WO2014086842, incorporated by reference in its entirety herein. Nucleotide sequences of the genomes can be obtained through gene data libraries available publicly and can allow for rational design and modifications of the pathways to enhance and improve product yield.

"Culture media" as described herein, can be a nutrient rich broth for the growth and maintenance of cells during their production phase. A yeast culture for maintaining and propagating various strains, can require specific formulations of complex media for use in cloning and protein expression, and can be appreciated by those of skill in the art. Commercially available culture media can be used from ThermoFisher for example. The media can be YPD broth or can have a yeast nitrogen base. Yeast can be grown in YPD or synthetic media at 30°C.

Lysogeny broth (LB) is typically used for bacterial cells. The bacterial cells used for growth of the enzymes and mogrosides can have antibiotic resistance to prevent the growth of other cells in the culture media and contamination. The cells can have an antibiotic gene cassettes for resistance to antibiotics such as chloramphenicol, penicillin, kanamycin and ampicillin, for example.

As described herien, a "fusion protein" is a protein created through the joining of two or more nucleic acid sequences that originally coded for a portion or entire amino acid sequence of separate proteins. For example, a fusion protein can contain a functional protein (e.g., an enzyme (including, but not limited to, cucurbitadienol synthase)) and one or more fusion domains. A fusion domain, as describe herein, can be a full length or a portion/fragment of a protein (e.g., a functional protein including but not limited to, an enzyme, a transcription factor, a toxin, and translation factor). The location of the one or more fusion domains in the fusion protein can vary. For example, the one or more fusion domains can be at the N- and/or C- terminal regions (e.g., N- and/or C- termini) of the fusion protein. The one or more fusion domains can also be at the central region of the fusion protein. The fusion domain is not required to be located at the terminus of the fusion protein. A fusion domain can be selected so as to confer a desired property. For example, a fusion domain may affect (e.g., increase or decrese) the enzymatic activity of an enzyme that it is fused to, or affect (e.g., incrase or decrease) the stability of a protein that it is fused to. A fusion domain may be a multimerizing (e.g., dimerizing and tetramerizing) domain and/or functional domains. In some embodiments, the fusion domain may enhance or decrease the multimerization of the protein that it is fused to. As a non-limiting example, a fusion protein can contain a full length protein A and a fusion domain fused to the N-terminal region and/or C-terminal region of the full length protein A. In some examples, a fusion protein contains a partial sequence of protein A and a fusion domain fused to the N-terminal region and/or C-terminal region (e.g., the N-terminus and C-terminus) of the partial sequnce of protein A. The fusion domain can be, for example, a portion or the entire sequence of protein A, or a portion or the entire sequence of a protein different from protein A. In some embodiments, one or more of the enzymes suitable for use in the methods, systems and compositions disclosed herein can be a fusion protein. In some embodiments, the fusion protein is encoded by a nucleic acid sequence having at least 70%, 80%, 90%, 95%, or 99% sequence identity to one of the nucleic acid sequences provided herein. In some embodiments, the fusion protein comprise an amino acid sequence having at least 70%, 80%, 90%, 95%, or 99% sequence identity to one of the amino acid sequences of SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 or SEQ ID NO: 25. In some embodiments, the fusion protein comprises an amino acid protein sequence having at least 80%, 90%, 95%, or 99% sequence identity to one of the amino acid sequences of SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 or SEQ ID NO: 25, and a fusion domain at N-, C-, or both terminal regions of the fusion protein. In some embodiments, the fusion protein comprises one of the amino acid protein sequences of SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 or SEQ ID NO: 25, and a fusion domain located at N-, C-, or both terminal regions of the fusion protein.

The length of the fusion domain can vary, for example, from 3, 4, 5, 6, 7, 8, 9, 10, 12, 15, 20, 25, 30, 40, 50, 60, 70, 80, 90, 100, 125, 150, 200, 300, 400, 500, 600, 700, 800, 900, 1000, 1100, 1200, 1300, 1400, 1500, or a range between any of these two numbers, amino acids. In some embodiments, the fusion domain is about 3, 4, 5, 5, 6, 7, 8, 9, 10, 12, 15, 20, 25, 30, 40, 50, or a range between any two of these numbers, amino acids in length. In some embodiments, the fusion domain is a substantial portion or the entire sequence of a functional protein (for example, an enzyme, a transcription factor, or a translation factor). In some embodiments, the fusion protein is a protein having cucurbitadienol synthase activity.

Optimizing cell growth and protein expression techniques in culture media are also contemplated. For growth in culture media, cells such as yeast can be sensitive to low pH (Narendranath et al., Appl Environ Microbiol. 2005 May; 71(5): 2239-2243; incorporated by reference in its entirety). During growth, yeast must maintain a constant intracellular pH. There are many enzymes functioning within the yeast cell during growth and metabolism. Each enzyme works best at its optimal pH, which is acidic because of the acidophilic nature of the yeast itself. When the extracellular pH deviates from the optimal level, the yeast cell needs to invest energy to either pump in or pump out hydrogen ions in order to maintain the optimal intracellular pH. As such media containing buffers to control for the pH would be optimal. Alternatively, the cells can also be transferred into a new media if the monitored pH is high.

Growth optimization of bacterial and yeast cells can also be achieved by the addition of nutrients and supplements into a culture media. Alternatively, the cultures can be grown in a fermenter designed for temperature, pH control and controlled aeration rates. Dissolved oxygen and nitrogen can flowed into the media as necessary.

The term "Operably linked" as used herein refers to functional linkage between a regulatory sequence and a heterologous nucleic acid sequence resulting in expression of the latter.

"Mogrosides" and "mogroside compounds" are used interchangeably herein and refer to a family of triterpene glycosides. Non-limiting exemplary examples of mogrosides include such as Mogroside V, Siamenoside I, Mogroside IV_{E}, Iso-mogroside V, Mogroside III_{E}, 11-Deoxy-mogroside V, 11-Oxo-mogroside V, Mogroside VI, Mogroside IV_{A}, Mogroside II_{A}, Mogroside II_{A1}, Mogroside II_{A2}, Mogroside I_{A}, 11-oxo-Mogroside VI, 11-oxo-Mogroside III_{E}, 11-oxo-Mogroside IV_{E}, Mogroside I_{E}, Mogrol, 11-oxo-mogrol, Mogroside II_{E}, Mogroside III_{A2}, and Mogroside III, which have been identified from the fruits of Siraitia grosvenorii (Swingle) that are responsible for the sweetness of the fruits. In the embodiments herein, mogroside intermediates can be used in the *in vivo, ex vivo,* or *in vitro* production of siamenoside I.

In some embodiments, a recombinant cell for producing siamenoside I further produces mogrosides and comprises genes encoding enzymes for the production of mogrosides. Recombinant cells capable of the production of mogrosides are further described in WO2014086842, incorporated by reference in its entirety herein. In some embodiments, the recombinant cell is grown in a media to allow expression of the enzymes and production of siamenoside I and mogroside intermediates. In some embodiments, siamenoside I is obtained by lysing the cell with shear force (i.e. French press cell or sonication) or by detergent lysing methods. In some embodiments, the cells are supplemented in the growth media with precursor molecules such as mogrol to boost production of siamenoside I

### Siamenoside I

As disclosed herein, siamenoside I is a compound having the structure of formula (I): 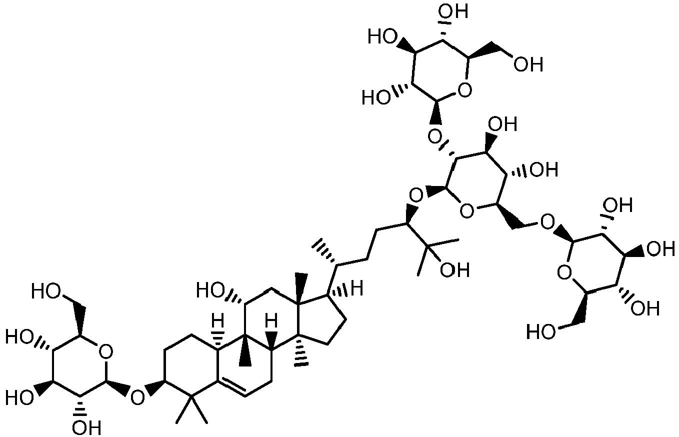 or a salt thereof.

Siamenoside I is a high-intensity sweetener the can be used in a wide variety of products in which a sweet taste is desired. Siamenoside I provides a low-calorie advantage to other sweeteners such as sucrose or fructose.

In some embodiments, siamenoside I is in an isolated and purified form. In some embodiments, siamenoside I is present in a composition in which siamenoside I is substantially purified.

In some embodiments, siamenoside I or salts thereof are isolated and is in solid form. In some embodiments, the solid form is amorphous. In some embodiments, the solid form is crystalline. In some embodiments, the compound is in the form of a lyophile. In some embodiments, siamenoside I is isolated and within a buffer.

The skilled artisan will recognize that some structures described herein may be resonance forms or tautomers of compounds that may be fairly represented by other chemical structures, even when kinetically; the artisan recognizes that such structures may only represent a very small portion of a sample of such compound(s). Such compounds are considered within the scope of the structures depicted, though such resonance forms or tautomers are not represented herein.

Isotopes may be present in siamenoside I. Each chemical element as represented in a compound structure may include any isotope of said element. For example, in a compound structure a hydrogen atom may be explicitly disclosed or understood to be present in the compound. At any position of the compound that a hydrogen atom may be present, the hydrogen atom can be any isotope of hydrogen, including but not limited to hydrogen-1 (protium) and hydrogen-2 (deuterium). Thus, reference herein to a compound encompasses all potential isotopic forms unless the context clearly dictates otherwise. In some embodiments, compounds described herein are enriched in one or more isotopes relative to the natural prevalence of such isotopes. In some embodiments, the compounds described herein are enriched in deuterium. In some embodiments, greater than 0.0312% of hydrogen atoms in the compounds described herein are deuterium. In some embodiments, greater than 0.05%, 0.08%, or 0.1% of hydrogen atoms in the compounds described herein are deuterium.

In some embodiments, siamenoside I is capable of forming acid and/or base salts by virtue of the presence of amino and/or carboxyl groups or groups similar thereto.

In some embodiments, siamenoside I is substantially isolated. In some embodiments, siamenoside I is substantially purified. In some embodiments, the compound is in the form of a lyophile. In some embodiments, the compound is crystalline. In some embodiments, the compound is amorphous.

### Production Compositions

In some embodiments, the production composition is in solid form, which may by crystalline or amorphous. In some embodiments, the composition is in particulate form. The solid form of the composition may be produced using any suitable technique, including but not limited to re-crystallization, filtration, solvent evaporation, grinding, milling, spray drying, spray agglomeration, fluid bed agglomeration, wet or dry granulation, and combinations thereof. In some embodiments, a flowable particulate composition is provided to facilitate use in further food manufacturing processes. In some such embodiments, a particle size between 50 µm and 300 µm, between 80 µm and 200 µm, or between 80 µm and 150 µm is generated.

Some embodiments provide a production composition comprising siamenoside I that is in solution form. For example, in some embodiments a solution produced by one of the production processes described herein is used without further purification. In some embodiments, the concentration of siamenoside I in the solution is greater than 300 ppm, 500 ppm, 800 ppm, 0.1%, 0.5%, 1%, 2%, 3%, 4%, 5%, 6%, 7%, 8%, 9%, 10%, 15%, or 20% by weight. In some embodiments, the concentration of all isomers of Mogroside I, Mogroside II, and Mogroside III is less than 5%, 3%, 2%, 1%, 0.5%, 0.3%, 0.1%, 800 ppm, 500 ppm, 200 ppm, or 100 ppm. In some embodiments, the concentration of one or more of Mogroside IIIE, 11-oxo-Mogroside IIIE, Mogroside IIIA2, Mogroside IE, Mogroside IIE, and 11-oxo-mogrol in the production composition is less than 5%, 3%, 2%, 1%, 0.5%, 0.3%, 0.1%, 800 ppm, 500 ppm, 200 ppm, 100 ppm, 50 ppm, 30 ppm, 20 ppm, 10 ppm, 5 ppm, 1 ppm, or 0.1 ppm of one or more of Mogroside IIIE, 11-oxo-Mogroside IIIE, Mogroside IIIA2, Mogroside IE, Mogroside IIE, and 11-oxo-mogrol. In some embodiments, the concentration of Mogroside IIIE is less than 5%, 3%, 2%, 1%, 0.5%, 0.3%, 0.1%, 800 ppm, 500 ppm, 200 ppm, 100 ppm, 50 ppm, 30 ppm, 20 ppm, 10 ppm, 5 ppm, 1 ppm, or 0.1 ppm. In some embodiments, the concentration of 11-oxo-Mogroside IIIE is less than 5%, 3%, 2%, 1%, 0.5%, 0.3%, 0.1%, 800 ppm, 500 ppm, 200 ppm, 100 ppm, 50 ppm, 30 ppm, 20 ppm, 10 ppm, 5 ppm, 1 ppm, or 0.1 ppm. In some embodiments, the concentration of 11-oxo-mogrol is less than 5%, 3%, 2%, 1%, 0.5%, 0.3%, 0.1%, 800 ppm, 500 ppm, 200 ppm, 100 ppm, 50 ppm, 30 ppm, 20 ppm, 10 ppm, 5 ppm, 1 ppm, or 0.1 ppm.

In some embodiments, the production composition contains none, or less than a certain amount, of undesirable compounds. In some embodiments, the composition contains, or does not contain, one or more isomers of Mogroside I, Mogroside II, and Mogroside III. In some embodiments, the composition contains a weight percent of less than 5%, 3%, 2%, 1%, 0.5%, 0.3%, 0.1%, 800 ppm, 500 ppm, 200 ppm, or 100 ppm of all isomers of Mogroside I, Mogroside II, and Mogroside III. In some embodiments, the composition contains, or does not contain, one or more of Mogroside IIIE, 11-oxo-Mogroside IIIE, Mogroside IIIA2, Mogroside IE, Mogroside IIE, and 11-oxo-mogrol. In some embodiments, the composition contains a weight percent of less than 5%, 3%, 2%, 1%, 0.5%, 0.3%, 0.1%, 800 ppm, 500 ppm, 200 ppm, or 100 ppm of one or more of Mogroside IIIE, 11-oxo-Mogroside IIIE, Mogroside IIIA2, Mogroside IE, Mogroside IIE, and 11-oxo-mogrol. In some embodiments, the composition contains a weight percent of less than 5%, 3%, 2%, 1%, 0.5%, 0.3%, 0.1%, 800 ppm, 500 ppm, 200 ppm, or 100 ppm of Mogroside IIIE. In some embodiments, the composition contains a weight percent of less than 5%, 3%, 2%, 1%, 0.5%, 0.3%, 0.1%, 800 ppm, 500 ppm, 200 ppm, or 100 ppm of 11-oxo-Mogroside IIIE. In some embodiments, the composition contains a weight percent of less than 5%, 3%, 2%, 1%, 0.5%, 0.3%, 0.1%, 800 ppm, 500 ppm, 200 ppm, or 100 ppm of 11-oxo-mogrol.

### Methods of Producing Siamenoside I and Intermediate Mogroside Compounds

The present invention relates to the preparation of siamenoside I using polypeptides having UDP-glycotransferase activity.

In some embodiments, siamenoside I is produced by contact of various starting and/or intermediate compounds with one or more enzymes. The contact can be *in vivo* (e.g., in a recombinant cell) or *in vitro.* The starting and intermediate compound for producing siamenoside I is a mogroside having a structure of formula (II) 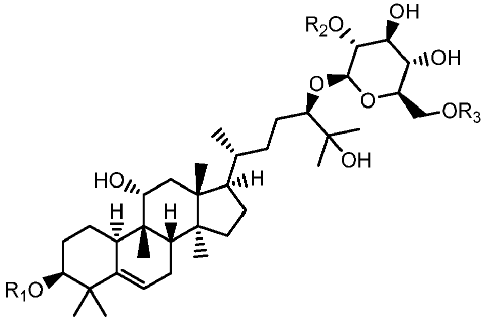 wherein R1, R2 and R3 is glucose or H.

In some embodiments, siamenoside I as disclosed herein is produced in recombinant host cells *in vivo* as described herein or by modification of these methods. Ways of modifying the methodology include, among others, temperature, solvent, reagents etc., known to those skilled in the art. The methods shown and described herein are illustrative only and are not intended, nor are they to be construed, to limit the scope of the claims in any manner whatsoever. Those skilled in the art will be able to recognize modifications of the disclosed methods and to devise alternate routes based on the disclosures herein; all such modifications and alternate routes are within the scope of the claims.

In some embodiments, siamenoside I disclosed herein is obtained by purification and/or isolation from a recombinant bacterial cell, yeast cell, plant cell, or insect cell. In some embodiments, the recombinant cell is from *Siraitia grosvenorii.* In some such embodiments, an extract obtained from *Siraitia grosvenorii* may be fractionated using a suitable purification technique. In some embodiments, the extract is fractionated using HPLC and the appropriate fraction is collected to obtain the desired compound in isolated and purified form.

In some embodiments, siamenoside I is produced by enzymatic modification of a compound isolated from *Siraitia grosvenorii.* For example, in some embodiments, a mogroside of formula (II) isolated from *Siraitia grosvenorii* is contacted with one or more enzymes to obtain the desired compounds. The contact can be *in vivo* (e.g., in a recombinant cell) or *in vitro.* The starting and intermediate compound for producing siamenoside I is a mogroside having a structure of formula (II) 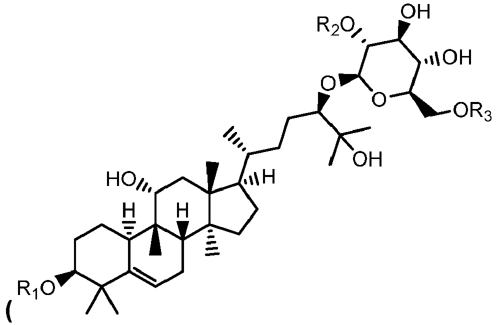 wherein R1, R2 and R3 is = glucose or H

In an embodiment of the invention the mogroside is mogroside III having a structure of formula (III)

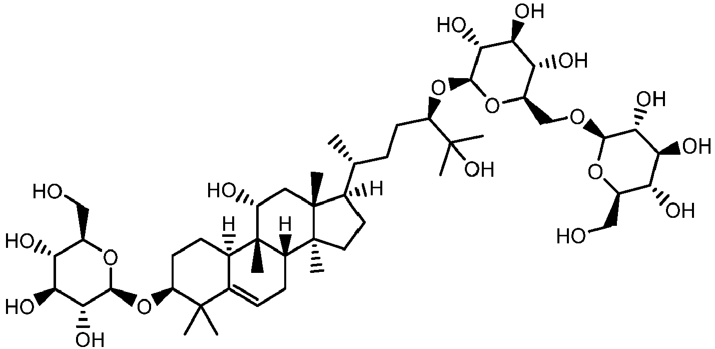

In an embodiment of the invention the mogroside is mogroside IIIA having a structure of formula (IV)

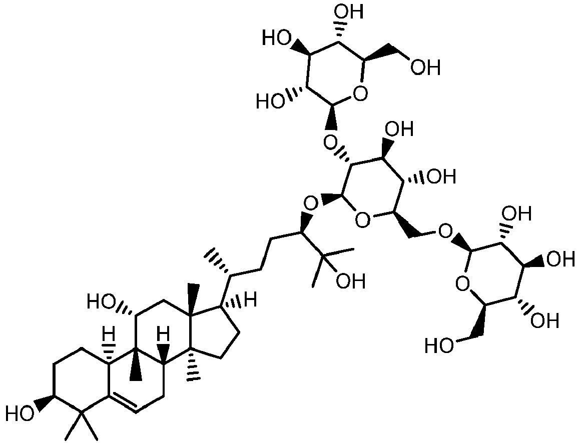

In an embodiment of the invention the mogroside is mogroside IIIE having a structure of formula (V)

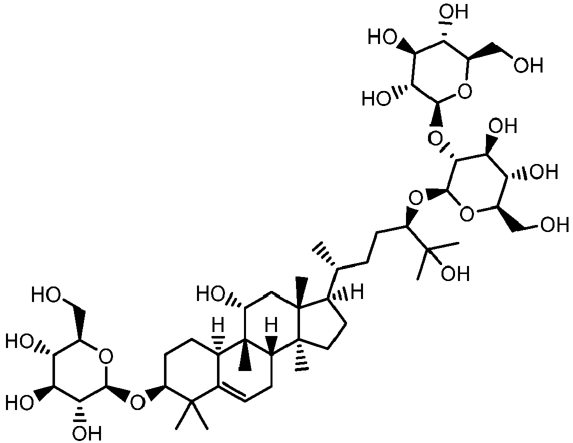

The mogorisides compounds used for the preparation of siamenoside I can be obtained commercially from various companes. Mogroside III has CAS Number 130567-83-8, Mogroside IIIA has CAS Number 88901-42-2 and Mogroside IIIE has CAS Number 88901-37-5.

Some embodiments provide a method of making siamenoside I, wherein the method comprises treating Mogroside III having a structure of formula (III) wherein the method requires contacting mogroside III, the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 1 or SEQ ID NO: 3.

Some embodiments provide a method of making siamenoside I, wherein the method comprises treating Mogroside IIIA having a structure of formula (III) wherein the method requires contacting mogroside IIIA, the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 5, SEQ ID NO: 7, or SEQ ID NO: 9, SEQ ID NO: 11, SEQ ID NO: 13, SEQ ID NO: 15, SEQ ID NO: 17 or SEQ ID NO: 19.

Some embodiments provide a method of making siamenoside I, wherein the method comprises treating Mogroside IIIE having a structure of formula (V) wherein the method requires contacting mogroside IIIE, the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 1, SEQ ID NO: 21 or SEQ ID NO: 23.

In some embodiments, the polypeptide having UDP-glycotransferase activity comprises an amino acid sequence set forth in SEQ ID NO: 21.

Also provided herein is a method of producing siamenoside I having the structure of formula (I): 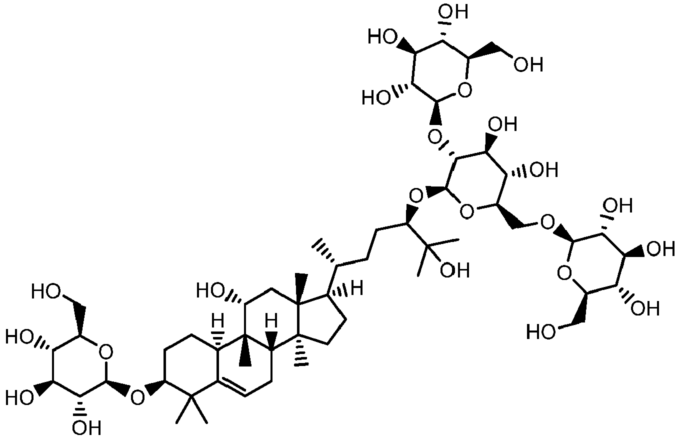 the method comprises contacting mogroside V having a structure of formula (VI) 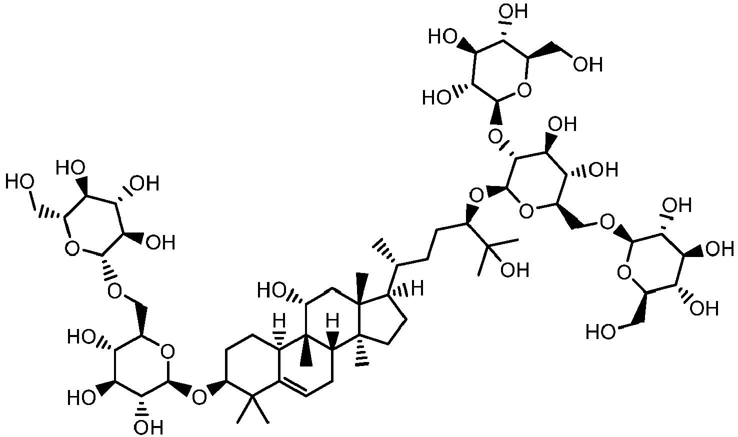 with a polypeptide having glucosidase activity.

In some embodiments, contacting a mogroside having a structure of formula (VI) with a polypeptide having glucosidase activity comprises contacting said mogroside having a structure of formula (VI) with a recombinant host cell that comprises a gene encoding the polypeptide having glucosidase activity.

In a preferred embodiment where the method requires contacting mogroside having a structure of formula (VI), the polypeptide having glucosidase activity comprises an amino acid sequence having at least 40%, 45%, 50%, 55%, 60%, 65%, 70%, 75%, 80%, 85%, 90%, 95%, 96%, 97%, 98% or 99% sequence identity to SEQ ID NO: 25.

The methods herein also include incorporating genes into the recombinant cells for producing intermediates such as pyruvate, acetyl-coa, citrate, and other TCA intermediates (Citric acid cycle). Intermediates can be further used to produce mogroside compounds for producing siamenoside I. Methods for increasing squalene content are described in Gruchattka *et al.* and Rodriguez et al. (PLoS One. 2015 Dec 23; 10(12; Microb Cell Fact. 2016 Mar 3; 15:48; incorporated by reference in their entireties herein).

Detailed information on the preparation of recombinant cells producing intermediates for the mogroside compounds of formula (II), which includes mogrosides III, IIIA and IIE, and compounds of formula (IV), which is mogroside V, are provided in the part. For example, WO2020096905 provides information on the genes, polypeptides and associated expression and recombinant methology required for the preparation of such recombinatnt cells. The contents of WO2020096905 are not intended to limit the scope of protection sought by the present invention but are incorporated herein by reference.

Expression of enzymes to produce oxidosqualene and diepoxysqualene are further contemplated. The use of enzymes to produce oxidosqualene and diepoxysqualene can be used to boost squalene synthesis by the way of squalene synthase and/or squalene epoxidase. For example, Su *et al.* describe the gene encoding SgSQS, a 417 amino acid protein from Siraitia grosvenorii for squalene synthase (Biotechnol Lett. 2017 Mar 28; incorporated by reference in its entirety herein). Genetically engineering the recombinant cell for expression of HMG CoA reductase is also useful for squalene synthesis (Appl Environ Microbiol. 1997 Sep; 63(9):3341-4.; Front Plant Sci. 2011 Jun 30; 2:25; FEBS J. 2008 Apr; 275(8):1852-9.; all incorporated by reference in their entireties herein.

Expression of enzymes to produce cucurbitadienol/epoxycucurbitadienol are also contemplated. Examples of curubitadienol synthases from C pepo, S grosvenorii, C sativus, C melo, C moschata, and C maxim are contemplated for engineering into the recombinant cells by a vector for expression. Oxidosqualene cyclases for titerpene biosynthesis is also contemplated for expression in the recombinant cell, which would lead to the cyclization of an acyclic substrate into various polycyclic triterpenes which can also be used as intermediates for the production of siamenoside I (Org Biomol Chem. 2015 Jul 14;13(26):7331-6; incorporated by reference in its entirety herein).

Expression of enzymes that display epoxide hydrolase activities to make hydroxy-cucurbitadienols are also contemplated. In some embodiments herein, the recombinant cells for the production of siamenoside I further comprises genes that encode enzymes that display epoxide hydrolase activities to make hydroxy-cucurbitadienols are provided. Such enzymes are provided in Itkin *et al.* which is incorporated by reference in its entirety herein.

The expression of enzymes in recombinant cells to that hydroxylate mogroside compounds to produce mogrol are also contemplated. These enzymes can include proteins of the CAZY family, UDP glycosyltransferases, CGTases, Glycotransferases, Dextransucrases, Cellulases, B-glucosidases, Transglucosidases, Pectinases, Dextranases, yeast and fungal hydrolyzing enzymes. Such enzymes can be used for example for hydrolyzing Mogroside V to Mogroside IIIE, in which Mogroside IIIE can be further processed to produce siamenoside I, for example *in vivo.*

In some embodiments, a mogrol precursor such as squalene or oxidosqualine, mogrol or mogroside is produced. The mogrol precursor can be used as a precursor in the production of siamenoside I. Squalene can be produced from famesyl pyrophosphate using a squalene synthase, and oxidosqualene can be produced from squalene using a squalene epoxidase. The squalene synthase can be, for example, squalene synthase from Gynostemma pentaphyllum (protein accession number C4P9M2), a cucurbitaceae family plant. The squalene synthase can also comprise a squalene synthase from *Arabidopsis thaliana* (protein accession number C4P9M3), *Brassica napus, Citrus macrophylla, Euphorbia tirucalli* (protein accession number B9WZW7), *Glycine max, Glycyrrhiza glabra* (protein accession number Q42760, Q42761 ), *Glycrrhiza uralensis* (protein accession number D6QX40, D6QX41 , D6QX42, D6QX43, D6QX44, D6QX45, D6QX47, D6QX39, D6QX55, D6QX38, D6QX53, D6QX37, D6QX35, B5AID5, B5AID4, B5AID3, C7EDD0, C6KE07, C6KE08, C7EDC9), *Lotusjaponicas* (protein accession number Q84LE3), *Medicago truncatula* (protein accession number Q8GSL6), *Pisum sativum, Ricinus communis* (protein accession number B9RHC3). Various squalene synthases have described in WO 2016/050890, the content of which is incorporated herein by reference in its entirety.

### Recombinant host cells

Any one of the enzymes disclosed herein can be produced *in vitro, ex vivo,* or *in vivo.* For example, a nucleic acid sequence encoding the UDP-glycotransferase can introduced to a host recombinant cell, for example in the form of an expression vector containing the coding nucleic acid sequence, *in vivo.* The expression vectors can be introduced into the host cell by, for example, standard transformation techniques (e.g., heat transformation) or by transfection. The expression systems can produce the enzymes for mogroside and siamenoside I production, in order to produce siamenoside I in the cell *in vivo.* Useful expression systems include, but are not limited to, bacterial, yeast and insect cell systems. For example, insect cell systems can be infected with a recombinant virus expression system for expression of the enzymes of interest. In some embodiments, the genes are codon optimized for expression in a particular cell. In some embodiments, the genes are operably linked to a promoter to drive transcription and translation of the enzyme protein. As described herein, codon optimization can be obtained, and the optimized sequence can then be engineered into a vector for transforming a recombinant host cell.

Expression vectors can further comprise transcription or translation regulatory sequences, coding sequences for transcription or translation factors, or various promoters (e.g., GPD1 promoters) and/or enhancers, to promote transcription of a gene of interest in yeast cells.

The recombinant cells as described herein are, in some embodiments, genetically modified to produce siamenoside I *in vivo.* Additionally, a cell can be fed a mogrol precursor or mogroside precursor during cell growth or after cell growth to boost rate of the production of a particular intermediate for the pathway for producing siamenoside I *in vivo.* The cell can be in suspension or immobilized. The cell can be in fermentation broth or in a reaction buffer. In some embodiments, a permeabilizing agent is used for transfer of a mogrol precursor or mogroside precursor into a cell. In some embodiments, a mogrol precursor or mogroside precursor can be provided in a purified form or as part of a composition or an extract.

The recombinant host cell can be, for example a plant, bivalve, fish, fungus, bacteria or mammalian cell. For example, the plant can be selected from Siraitia, Momordica, Gynostemma, Cucurbita, Cucumis, Arabidopsis, Artemisia, Stevia, Panax, Withania, Euphorbia, Medicago, Chlorophytum, Eleutherococcus, Aralia, Morus, Medicago, Betula, Astragalus, Jatropha, Camellia, Hypholoma, Aspergillus, Solanum, Huperzia, Pseudostellaria, Corchorus, Hedera, Marchantia, and Morus. The fungus can be selected from Trichophyton, Sanghuangporus, Taiwanofungus, Moniliophthora, Marssonina, Diplodia, Lentinula, Xanthophyllomyces, Pochonia, Colletotrichum, Diaporthe, Histoplasma, Coccidioides, Histoplasma, Sanghuangporus, Aureobasidium, Pochonia, Penicillium, Sporothrix, Metarhizium, Aspergillus, Yarrowia, and Lipomyces. In some embodiments, the fungus is *Aspergillus nidulans, Yarrowia lipolytica,* or *Rhodosporin toruloides.* In some embodiments, the recombinant host cell is a yeast cell. In some embodiments, the yeast is selected from Candida, Sacccharaomyces, Saccharomycotina, Taphrinomycotina, Schizosaccharomycetes, Komagataella, Basidiomycota, Agaricomycotina, Tremellomycetes, Pucciniomycotina, Aureobasidium, Coniochaeta, Rhodosporidium, Yarrowia, and Microboryomycetes. In some embodiments, the bacteria is selected from Frankia, Actinobacteria, Streptomyces, and Enterococcus. In some embodiments, the bacteria is *Enterococcus faecalis.*

In some embodiments, the recombinant genes are codon optimized for expression in a bacterial, mammalian, plant, fungal or insect cell. In some embodiments, one or more of genes comprises a functional mutation to increased activity of the encoded enzyme. In some embodiments, cultivating the recombinant host cell comprises monitoring the cultivating for pH, dissolved oxygen level, nitrogen level, or a combination thereof of the cultivating conditions.

The recombinant host cell can be, for examole, a plant, bivalve, fish, fungus, bacteria or mammalian cell. For example, the plant is selected from Siraitia, Momordica, Gynostemma, Cucurbita, Cucumis, Arabidopsis, Artemisia, Stevia, Panax, Withania, Euphorbia, Medicago, Chlorophytum, Eleutherococcus, Aralia, Morus, Medicago, Betula, Astragalus, Jatropha, Camellia, Hypholoma, Aspergillus, Solanum, Huperzia, Pseudostellaria, Corchorus, Hedera, Marchantia, and Morus. In some embodiments, fungus is selected from Trichophyton, Sanghuangporus, Taiwanofungus, Moniliophthora, Marssonina, Diplodia, Lentinula, Xanthophyllomyces, Pochonia, Colletotrichum, Diaporthe, Histoplasma, Coccidioides, Histoplasma, Sanghuangporus, Aureobasidium, Pochonia, Penicillium, Sporothrix, Metarhizium, Aspergillus, Yarrowia, and Lipomyces. In some embodiments, the fungus is *Aspergillus nidulans, Yarrowia lipolytica,* or *Rhodosporin toruloides.* In some embodiments, the recombinant host cell is a yeast cell. In some embodiments, the yeast is selected from Candida, Sacccharaomyces, Saccharomycotina, Taphrinomycotina, Schizosaccharomycetes, Komagataella, Basidiomycota, Agaricomycotina, Tremellomycetes, Pucciniomycotina, Aureobasidium, Coniochaeta, Rhodosporidium, and Microboryomycetes. In some embodiments, the bacteria is selected from Frankia, Actinobacteria, Streptomyces, Enterococcus, In some embodiments, the bacteria is *Enterococcus faecalis.* In some embodiments, one or more of the first, second third, fourth, fifth, sixth, seventh, eighth, ninth, and tenth genes has been codon optimized for expression in a bacterial, mammalian, plant, fungal or insect cell. In some embodiments, one or more of the first, second third, fourth, fifth, sixth, seventh, eighth, ninth, and tenth genes comprises a functional mutation to increased activity of the encoded enzyme. In some embodiments, cultivating the recombinant host cell comprises monitoring the cultivating for pH, dissolved oxygen level, nitrogen level, or a combination thereof of the cultivating conditions. In some embodiments, the method comprises isolating siamenoside I. In some embodiments, isolating siamenoside I comprises lysing the recombinant host cell. In some embodiments, isolating siamenoside I comprises isolating siamenoside I from the culture medium. In some embodiments, the method comprises purifying siamenoside I. In some embodiments, purifying siamenoside I comprises HPLC, solid phase extraction or a combination thereof. In some embodiments, the purifying comprises harvesting the recombinant cells, saving the supernatant and lysing the cells. In some embodiments, the lysing comprises subjecting the cells to shear force or detergent washes thereby obtaining a lysate. In some embodiments, the shear force is from a sonication method, french pressurized cells, or beads. In some embodiments, the lysate is subjected to filtering and purification steps. In some embodiments, the lysate is filtered and purified by solid phase extraction.

In some embodiments, siamenoside I having the structure of formula (I) 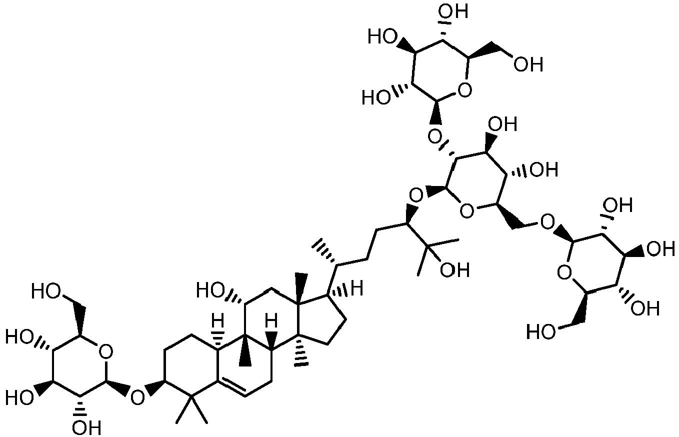 is provided, wherein the compound is produced by the method of any one of the alternative methods provided herein.

In some embodiments, a cell lysate comprising siamenoside I is provided.

In some embodiments, a recombinant cell comprising: siamenoside I is provided, and a gene encoding polypeptide having UDP-glycotransferase activity.

In some embodiments, the gene is a heterologous gene to the recombinant cell.

In some embodiments, a recombinant host cell or a non-human host organism comprising a polypeptide having UDP-glycotransferase activity or glucosidase is provided, comprising an acid molecule encoding a UDP-glycotransferase or glucosidase activity, wherein the polypeptide comprises an amino acid sequence having at least 40%, 45%, 50%, 55%, 60%, 65%, 70%, 75%, 80%, 85%, 90%, 95%, 96%, 97%, 98% or 99% sequence identity to SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 or SEQ ID NO: 25.

In some embodiments, the cell is a mammalian, bacterial, fungal, or insect cell. In some embodiments, the cell is a yeast cell. Non-limiting examples of the yeast include Candida, Sacccharaomyces, Saccharomycotina, Taphrinomycotina, Schizosaccharomycetes, Komagataella, Basidiomycota, Agaricomycotina, Tremellomycetes, Pucciniomycotina, Aureobasidium, Coniochaeta, and Microboryomycetes. In some embodiments, the plant is selected from the group consisting of Siraitia, Momordica, Gynostemma, Cucurbita, Cucumis, Arabidopsis, Artemisia, Stevia, Panax, Withania, Euphorbia, Medicago, Chlorophytum, Eleutherococcus, Aralia, Morus, Medicago, Betula, Astragalus, Jatropha, Camellia, Hypholoma, Aspergillus, Solanum, Huperzia, Pseudostellaria, Corchorus, Hedera, Marchantia, and Morus. In some embodiments, the fungus is Trichophyton, Sanghuangporus, Taiwanofungus, Moniliophthora, Marssonina, Diplodia, Lentinula, Xanthophyllomyces, Pochonia, Colletotrichum, Diaporthe, Histoplasma, Coccidioides, Histoplasma, Sanghuangporus, Aureobasidium, Pochonia, Penicillium, Sporothrix, or Metarhizium.

In some embodiments, DNA can be obtained through gene synthesis. This can be performed by either through Genescript or IDT, for example. DNA can be cloned through standard molecular biology techniques into an overexpression vector such as: pQE1, pGEX-4t3, pDest-17, pET series, pFASTBAC, for example. *E. coli* host strains can be used to produce enzyme (i.e., Top10 or BL21 series +/- codon plus) using 1mM IPTG for induction at OD600 of 1. *E. coli* strains can be propagated at 37C, 250 rpm and switched to room temperature or 30C (150rpm) during induction. When indicated, some enzymes can also be expressed through SF9 insect cell lines using pFASTBAC and optimized MOI. Crude extract containing enzymes can be generated through sonication and used for the reactions described herein. All UDP-glycosyltransferase reactions contain sucrose synthase, and can be obtained from *A. thaliana* via gene synthesis and expressed in *E. coli.*

### Purification of mogroside compounds

Some embodiments comprise isolating siamenoside I. In some embodiments, isolating siamenoside I comprises lysing the recombinant host cell. In some embodiments, isolating siamenoside I comprises isolating siamenoside I from the culture medium. In some embodiments, the method further comprises purifying siamenoside I. In some embodiments, purifying siamenoside I comprises HPLC, solid phase extraction or a combination thereof. In some embodiments, the purifying comprises harvesting the recombinant cells, saving the supernatant and lysing the cells. In some embodiments, the lysing comprises subjecting the cells to shear force or detergent washes thereby obtaining a lysate. In some embodiments, the shear force is from a sonication method, french pressurized cells, or beads. In some embodiments, the lysate is subjected to filtering and purification steps. In some embodiments, the lysate is filtered and purified by solid phase extraction. The lysate can then be filtered and treated with ammonium sulfate to remove proteins, and fractionated on a C18 HPLC (5 X 10 cm Atlantis prep T3 OBD column, 5 um, Waters) and by injections using an A/B gradient (A = water B = acetonitrile) of 10 → 30% B over 30 minutes, with a 95% B wash, followed by re-equilibration at 1% (total run time = 42 minutes). The runs can be collected in tared tubes (12 fractions/plate, 3 plates per run) at 30 mL/fraction. The lysate can also be centrifuged to remove solids and particulate matter. Plates can then be dried in the Genevac HT12/HT24. The desired compound is expected to be eluted in Fraction 21 along with other isomers. The pooled Fractions can be further fractionated in 47 runs on fluoro-phenyl HPLC column (3 X 10 cm, Xselect fluoro-phenyl OBD column, 5 um, Waters) using an A/B gradient (A = water, B = acetonitrile) of 15 → 30% B over 35 minutes, with a 95% B wash, followed by re-equilibration at 15% (total run time = 45 minutes). Each run was collected in 12 tared tubes (12 fractions/plate, 1 plate per run) at 30 mL/fraction. Fractions containing the desired peak with the desired purity can be pooled based on UPLC analysis and dried under reduced pressure to give a whitish powdery solid. The pure compound can be resuspended/dissolved in 10 mL of water and lyophilized to obtain at least a 95% purity.

For purification of siamenoside I in some embodiments, the compound can be purified by solid phase extraction, which may remove the need to HPLC. siamenoside I can be purified, for example, to or to about 70%, 80%, 90%, 95%, 98%, 99%, or 100% purity or any level of purity within a range described by any two aforementioned value.s In some embodiments, compound 1 that is purified by solid phase extraction is, or is substantially, identical to the HPLC purified material. In some embodiments, the method comprises fractionating lysate from a recombinant cell on an HPLC column and collecting an eluted fraction comprising siamenoside I

### Fermentation

Host cells can be fermented as described herein for the production of siamenoside I. This can also include methods that occur with or without air and can be carried out in an anaerobic environment, for example. The whole cells (e.g., recombinant host cells) may be in fermentation broth or in a reaction buffer.

Monk fruit (*Siraitia grosvenorii*) extract can also be used to contact the cells in order to produce siamenoside I. In some embodiments, a method of producing siamenoside I is provided. The method can comprise contacting monk fruit extract with a first enzyme capable of catalyzing production of siamenoside I from a mogroside having a structure of formula (II) or (VI).

In some embodiments, the contacting comprises contacting the mogrol fruit extract with a recombinant host cell that comprises gene encoding a polypeptide having UDP-glycotransferase or glucosidase activity. In some embodiments, the gene is heterologous to the recombinant host cell. In some embodiments, the mogrol fruit extract contacts with the polypeptide having UDP-glycotransferase or glucosidase activity in a recombinant host cell that comprises a the polypeptide having UDP-glycotransferase or glucosidase activity.

In general, compounds as disclosed and described herein, individually or in combination, can be provided in a composition, such as, e.g., an ingestible composition. In one embodiment, compounds as disclosed and described herein, individually or in combination, can provide a sweet flavor to an ingestible composition. In other embodiments, the compounds disclosed and described herein, individually or in combination, can act as a sweet flavor enhancer to enhance the sweeteness of another sweetener. In other embodiments, the compounds disclosed herein impart a more sugar-like temporal profile and/or flavor profile to a sweetener composition by combining one or more of the compounds as disclosed and described herein with one or more other sweeteners in the sweetener composition. In another embodiment, compounds as disclosed and described herein, individually or in combination, can increase or enhance the sweet taste of a composition by contacting the composition thereof with the compounds as disclosed and described herein to form a modified composition. In another embodiment, compounds as disclosed and described herein, individually or in combination, can be in a composition that modulates the sweet receptors and/or their ligands expressed in the body other than in the taste buds.

As used herein, an "ingestible composition" includes any composition that, either alone or together with another substance, is suitable to be taken by mouth whether intended for consumption or not. The ingestible composition includes both "food or beverage products" and "non-edible products". By "Food or beverage products", it is meant any edible product intended for consumption by humans or animals, including solids, semi-solids, or liquids (e.g., beverages) and includes functional food products (e.g., any fresh or processed food claimed to have a health-promoting and/or disease-preventing properties beyond the basic nutritional function of supplying nutrients). The term "non-food or beverage products" or "noncomestible composition" includes any product or composition that can be taken into the mouth by humans or animals for purposes other than consumption or as food or beverage. For example, the non-food or beverage product or noncomestible composition includes supplements, nutraceuticals, pharmaceutical and over the counter medications, oral care products such as dentifrices and mouthwashes, and chewing gum.

In some aspects, the compopsitions dislosed herein further comprise at least one additional sweetener and/or sweet modifier. The at least one additional sweetener and/or sweet modifier may be an artificial sweetener and/or sweet modifier, or, alternatively, a natural sweetener and/or sweet modifier. The at least one additional sweetener and/or sweet modifier may be selected from the group consisting of: abiziasaponin, abrusosides, in particular abrusoside A, abrusoside B, abrusoside C, abrusoside D, acesulfame potassium, advantame, albiziasaponin, alitame, aspartame, superaspartame, bayunosides, in particular bayunoside 1, bayunoside 2, brazzein, bryoside, bryonoside, bryonodulcoside, carnosifloside, carrelame, curculin, cyanin, chlorogenic acid, cyclamates and its salts, cyclocaryoside I, dihydroquercetin-3 -acetate, dihydroflavenol, dulcoside, gaudichaudioside, glycyrrhizin, glycyrrhetin acid, gypenoside, hematoxylin, isomogrosides, in particular iso-mogroside V, lugduname, magap, mabinlins, micraculin, mogrosides (lo han guo), in particular mogroside IV and mogroside V, monatin and its derivatives, monellin, mukurozioside, naringin dihydrochalcone (NarDHC), neohesperidin dihydrochalcone (NHDC), neotame, osladin, pentadin, periandrin I-V, perillartine, D-phenylalanine, phlomisosides, in particular phlomisoside 1, phlomisoside2, phlomisoside 3, phlomisoside 4, phloridzin, phyllodulcin, polpodiosides, polypodoside A, pterocaryosides, rebaudiosides, in particular rebaudioside A, rebaudioside B, rebaudioside C, rebaudioside D, rebaudioside E, rebaudioside F, rebaudioside G, rebaudioside H, rebaudioside M, rubusosides, saccharin and its salts and derivatives, scandenoside, selligueanin A, siamenosides, in particular siamenoside I, stevia, steviolbioside, stevioside and other steviol glycosides, strogines, in particular strogin 1, strogin 2, strogin 4, suavioside A, suavioside B, suavioside G, suavioside H, suavioside I, suavioside J, sucralose, sucronate, sucrooctate, talin, telosmoside A15, thaumatin, in particular thaumatin I and II, trans-anethol, trans- cinnamaldehyde, trilobatin, D-tryptophane, erythritol, galactitol, hydrogenated starch syrups including maltitol and sorbitol syrups, inositols, isomalt, lactitol, maltitol, mannitol, xylitol, arabinose, dextrin, dextrose, fructose, high fructose corn syrup, fructooligosaccharides, fructooligosaccharide syrups, galactose, galactooligosaccharides, glucose, glucose and (hydrogenated) starch syrups/hydrolysates, isomaltulose, lactose, hydrolysed lactose, maltose, mannose, rhamnose, ribose, sucrose, tagatose, trehalose and xylose.

### Compositions comprising siamenoside I

Also disclosed herein include compositions, e.g., ingestible compositions, comprising siamenoside I. In some embodiments, an ingestible composition can be a beverage. For example, the beverage can be selected from the group consisting of enhanced sparkling beverages, colas, lemon-lime flavored sparkling beverages, orange flavored sparkling beverages, grape flavored sparkling beverages, strawberry flavored sparkling beverages, pineapple flavored sparkling beverages, ginger-ales, root beers, fruit juices, fruit-flavored juices, juice drinks, nectars, vegetable juices, vegetable-flavored juices, sports drinks, energy drinks, enhanced water drinks, enhanced water with vitamins, near water drinks, coconut waters, tea type drinks, coffees, cocoa drinks, beverages containing milk components, beverages containing cereal extracts and smoothies. In some embodiments, the beverage can be a soft drink.

An "ingestibly acceptable ingredient" is a substance that is suitable to be taken by mouth and can be combined with a compound described herein to form an ingestible composition. The ingestibly acceptable ingredient may be in any form depending on the intended use of a product, e.g., solid, semi-solid, liquid, paste, gel, lotion, cream, foamy material, suspension, solution, or any combinations thereof (such as a liquid containing solid contents). The ingestibly acceptable ingredient may be artificial or natural. Ingestibly acceptable ingredients includes many common food ingredients, such as water at neutral, acidic, or basic pH, fruit or vegetable juices, vinegar, marinades, beer, wine, natural water/fat emulsions such as milk or condensed milk, edible oils and shortenings, fatty acids and their alkyl esters, low molecular weight oligomers of propylene glycol, glyceryl esters of fatty acids, and dispersions or emulsions of such hydrophobic substances in aqueous media, salts such as sodium chloride, wheat flours, solvents such as ethanol, solid edible diluents such as vegetable powders or flours, or other liquid vehicles; dispersion or suspension aids; surface active agents; isotonic agents; thickening or emulsifying agents, preservatives; solid binders; lubricants and the like.

Additional ingestibly acceptable ingredients include acids, including but are not limited to, citric acid, phosphoric acid, ascorbic acid, sodium acid sulfate, lactic acid, or tartaric acid; bitter ingredients, including, for example caffeine, quinine, green tea, catechins, polyphenols, green robusta coffee extract, green coffee extract, whey protein isolate, or potassium chloride; coloring agents, including, for example caramel color, Red #40, Yellow #5, Yellow #6, Blue #1, Red #3, purple carrot, black carrot juice, purple sweet potato, vegetable juice, fruit juice, beta carotene, turmeric curcumin, or titanium dioxide; preservatives, including, for example sodium benzoate, potassium benzoate, potassium sorbate, sodium metabisulfate, sorbic acid, or benzoic acid; antioxidants including, for example ascorbic acid, calcium disodium EDTA, alpha tocopherols, mixed tocopherols, rosemary extract, grape seed extract, resveratrol, or sodium hexametaphosphate; vitamins or functional ingredients including, for example resveratrol, Co-Q10, omega 3 fatty acids, theanine, choline chloride (citocoline), fibersol, inulin (chicory root), taurine, panax ginseng extract, guanana extract, ginger extract, L-phenylalanine, L-carnitine, L-tartrate, D-glucoronolactone, inositol, bioflavonoids, Echinacea, ginko biloba, yerba mate, flax seed oil, garcinia cambogia rind extract, white tea extract, ribose, milk thistle extract, grape seed extract, pyrodixine HCl (vitamin B6), cyanoobalamin (vitamin B12), niacinamide (vitamin B3), biotin, calcium lactate, calcium pantothenate (pantothenic acid), calcium phosphate, calcium carbonate, chromium chloride, chromium polynicotinate, cupric sulfate, folic acid, ferric pyrophosphate, iron, magnesium lactate, magnesium carbonate, magnesium sulfate, monopotassium phosphate, monosodium phosphate, phosphorus, potassium iodide, potassium phosphate, riboflavin, sodium sulfate, sodium gluconate, sodium polyphosphate, sodium bicarbonate, thiamine mononitrate, vitamin D3, vitamin A palmitate, zinc gluconate, zinc lactate, or zinc sulphate; clouding agents, including, for example ester gun, brominated vegetable oil (BVO), or sucrose acetate isobutyrate (SAIB); buffers, including, for example sodium citrate, potassium citrate, or salt; flavors, including, for example propylene glycol, ethyl alcohol, glycerine, gum Arabic (gum acacia), maltodextrin, modified corn starch, dextrose, natural flavor, natural flavor with other natural flavors (natural flavor WONF), natural and artificial flavors, artificial flavor, silicon dioxide, magnesium carbonate, or tricalcium phosphate; and stabilizers, including, for example pectin, xanthan gum, carboxylmethylcellulose (CMC), polysorbate 60, polysorbate 80, medium chain triglycerides, cellulose gel, cellulose gum, sodium caseinate, modified food starch, gum Arabic (gum acacia), or carrageenan.

### EXAMPLES

Some aspects of the embodiments discussed above are disclosed in further detail in the following examples, which are not in any way intended to limit the scope of the present disclosure.

### Example 1: Glycosylation of Mogroside IIIE to Siamenoside I

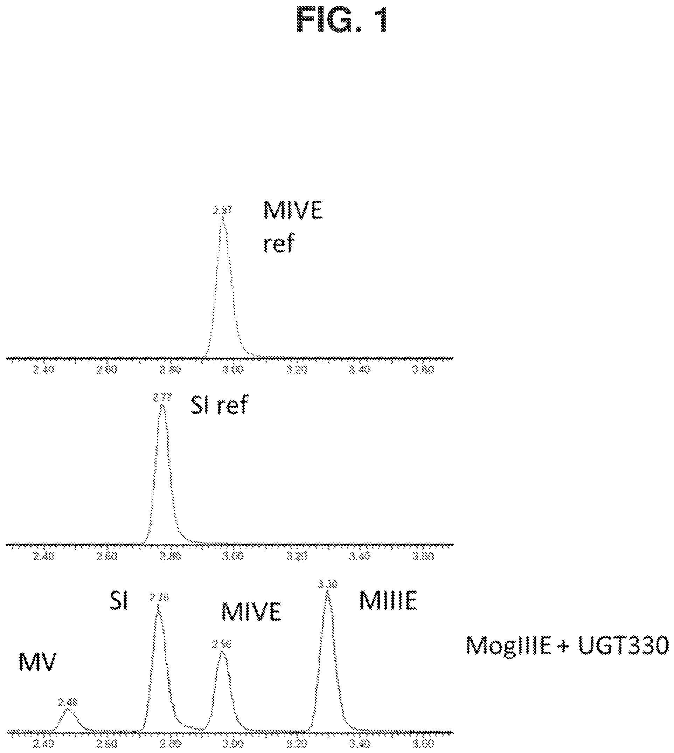

The use of UDP-glycotransferase 330 [6] led to the reaction products Siamenoside I, Mogroside IVE and Mogroside V (Figure 1). The gene from *Sesamum indicum,* was overexpressed in *Ecoli* (BL21-RIL) using overexpression vector pET-28a. For the reaction, I mg/ml of Mogroside **IIIE** was reacted with 250ul crude extract containing UDP-glycotransferase 330, 2ul crude extract containing sucrose synthase, 3mM UDP-Glucose, Ix M221 protease inhibitor, 200mM sucrose, 0.5mg/ml spectinomycin, 5mM MgCl, 5mM KCI, in 0.1M Tris-HCl pH7.0, incubated at 30°C. The sample below in the figure below was taken after 4hrs. The reactions were analyzed by UPLC (fluorophenyl column, 15 to 30, 6 minutes). The protein and DNA sequence of UGT330 is provided in SEQ ID NO: 21 and SEQ ID NO: 22, respectively.

### Example 2: Hydrolysis of Mogroside V to Siamenoside I

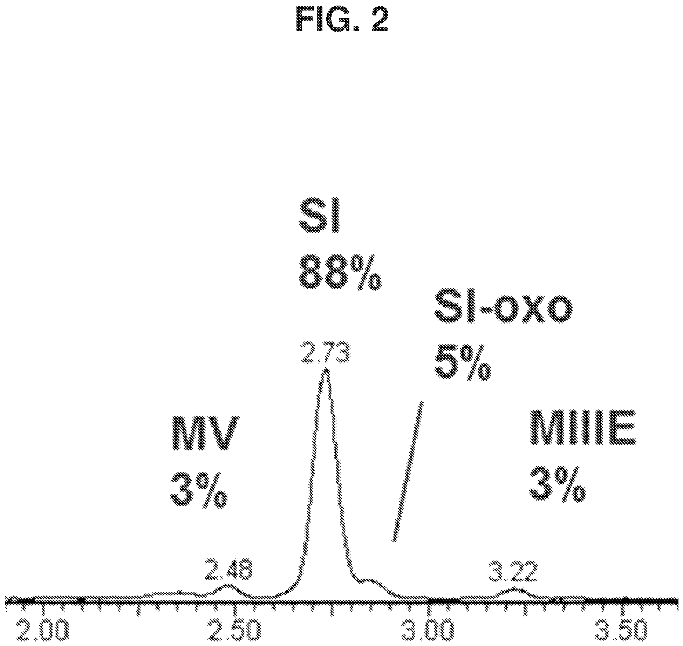

A polypeptide having the amino acid sequence provided in SEQ ID NO 25 was produced by S. *cerevisiae (Δexg1*) by cloning nucleic acid SEQ ID NO 26 into pESC-Ura and transformed into S. *cerevisiae* (*Δexg1*). The strain was grown in 10 mL of SC-Ura solution and incubated at 30°C for 2 days at 250 rpm. 10 mL of the seed culture was used to inoculate 1 L of SC-Ura solution with 100 g/L Luo Han Guo (LHG) extract without glucose supplementation in 2.8 L non-baffled Fernbach flask at 125 rpm. After 3 days, the supernatant was collected for HPLC and majority of Mogroside V was hydrolyzed to mostly Siamenoside I (Figure 2, Seql25: Protein, Seql26: DNA). The hydrolysis reactions were analyzed by UPLC (fluorophenyl column, 15 to 30, 6 minutes).

### Example 3: Growth of modified S. cerevisiae overproducing polypeptide SEQ ID NO 25 on mogroside extract without glucose.

*S. cerevisiae* (*Δexg1*) with overexpression of hydrolytic enzyme provided by the polypeptide having the amino acid sequence provided in SEQ ID NO 25 was grown in 10 mL of SC-Ura solution and incubated at 30°C for 2 days at 250 rpm. 10 mL of the seed culture was used to inoculate 1 L of SC-Ura solution with 100 g/L Luo Han Guo (LHG) extract without glucose supplementation in 2.8 L non-baffled Fernbach flask at 125 rpm. For controls, *S. cerevisiae* parent strain (BY4741) and strain (Δ*exg1*), were incubated in the same conditions above but with SC-complete. S. cerevisiae strains without overexpression of SEQ ID NO 25, were either unable to grow (Δ*exg1*), or hampered in growth (parent) when replacing glucose with mogroside extract.

### SEQUENCE LISTING

SEQ ID NO 1 *Siraitia grosvenorii* 
MDAQRGHTTTILMFPWLGYGHLSAFLELAKSLSRRNFHIYFCSTSVNLDAIKPKLPSSS
SSDSIQLVELCLPSSPDQLPPHLHTTNALPPHLMPTLHQAFSMAAQHFAAILHTLAPHLL
IYDSFQPWAPQLASSLNIPAINFNTTGASVLTRMLHATHYPSSKFPISEFVLHDYWKAM
YSAAGGAVTKKDHKIGETLANCLHASCSVILINSFRELEEKYMDYLSVLLNKKVVPVGPL
VYEPNQDGEDEGYSSIKNWLDKKEPSSTVFVSFGSEYFPSKEEMEEIAHGLEASEVHF
IWVVRFPQGDNTSAIEDALPKGFLERVGERGMVVKGWAPQAKILKHWSTGGFVSHCG
WNSVMESMMFGVPIIGVPMHLDQPFNAGLAEEAGVGVEAKRDSDGKIQREEVAKSIK
EVVIEKTREDVRKKAREMGEILRSKGDEKIDELVAEISLLRKKAPCSI.
SEQ ID NO 2: Nucleotide sequence encoding for SEQ ID NO 1 
ATGGACGCTCAGCGTGGTCACACTACTACTATCCTGATGTTCCCCTGGCTCGGTTA
CGGTCACCTGTCCGCTTTCTTGGAGCTGGCTAAGTCCCTGTCTCGTCGTAACTTCC
ACATCTACTTTTGCTCCACCTCCGTGAACCTGGACGCTATCAAGCCCAAGCTGCCT
TCCTCCTCATCCTCCGACTCTATCCAGCTGGTCGAGCTGTGCCTGCCTTCTTCACC
TGACCAGCTGCCTCCACACTTGCACACCACTAACGCTCTGCCACCTCACCTGATGC
CTACTCTGCACCAGGCTTTCTCCATGGCTGCTCAGCACTTCGCTGCTATCCTGCAC
ACCCTGGCTCCTCACCTGTTGATCTACGACTCCTTCCAGCCTTGGGCTCCCCAGCT
GGCTTCCTCTTTGAACATCCCCGCTATCAACTTCAACACCACCGGTGCTTCCGTGC
TGACCCGTATGTTGCACGCTACTCACTACCCCTCCAGCAAGTTCCCCATCTCCGAG
TTCGTGCTGCACGACTACTGGAAGGCTATGTACTCCGCTGCTGGTGGTGCTGTGA
CCAAGAAAGACCACAAGATCGGCGAGACTCTGGCTAACTGCCTGCACGCTTCCTG
CTCCGTGATCCTGATCAACTCCTTCCGCGAGCTGGAAGAGAAGTACATGGACTAC
CTGTCCGTGCTGCTGAACAAAAAGGTGGTGCCCGTGGGTCCCCTGGTGTACGAAC
CTAATCAGGACGGCGAGGACGAGGGCTACTCCTCCATTAAGAACTGGCTGGACAA
GAAAGAACCCTCCTCCACCGTGTTCGTGTCCTTCGGTTCCGAGTACTTCCCATCCA
AAGAAGAGATGGAAGAGATCGCTCACGGCTTGGAGGCTTCCGAGGTGCACTTCAT
CTGGGTCGTGCGTTTCCCTCAGGGCGACAACACCTCTGCTATCGAGGACGCTCTC
 
CCCAAGGGTTTCCTGGAAAGAGTGGGAGAGCGCGGCATGGTGGTCAAAGGATGG
GCTCCTCAGGCTAAGATCCTGAAGCACTGGTCCACCGGCGGTTTCGTGTCTCACT
GCGGATGGAACTCCGTGATGGAATCCATGATGTTCGGTGTCCCCATCATCGGCGT
GCCCATGCACTTGGACCAACCTTTCAACGCTGGCCTGGCTGAAGAGGCTGGTGTC
GGTGTAGAAGCTAAGCGCGACTCCGACGGAAAGATCCAGCGTGAAGAAGTCGCC
AAGTCCATCAAAGAGGTGGTCATCGAAAAGACCCGCGAGGACGTGCGTAAAAAGG
CTCGCGAGATGGGCGAGATCCTGCGTTCTAAGGGCGACGAGAAGATCGACGAGC
TGGTGGCTGAGATCTCCCTGCTGAGGAAGAAGGCTCCCTGCTCCATCGCGGCCG
CTTTCGAATCTAGAGCCTGCAGTCTCGAGGCATGCGGTACCAAGCTTGTCGAGAA
GTACTAG
SEQ ID NO 3: *Siraitia grosvenorii* 
MDAQRGHTTTILMLPWVGYGHLLPFLELAKSLSRRKLFHIYFCSTSVSLDAIKPKLPPSI
SSDDSIQLVELRLPSSPELPPHLHTTNGLPSHLMPALHQAFVMAAQHFQVILQTLAPHL
LIYDILQPWAPQVASSLNIPAINFSTTGASMLSRTLHPTHYPSSKFPISEFVLHNHWRAM
YTTADGALTEEGHKIEETLANCLHTSCGVVLVNSFRELETKYIDYLSVLLNKKVVPVGPL
VYEPNQEGEDEGYSSIKNWLDKKEPSSTVFVSFGTEYFPSKEEMEEIAYGLELSEVNFI
WVLRFPQGDSTSTIEDALPKGFLERAGERAMVVKGWAPQAKILKHWSTGGLVSHCG
WNSMMEGMMFGVPIIAVPMHLDQPFNAGLLEEAGVGVEAKRGSDGKIQREEVAKSIK
EVVIEKTREDVRKKAREMGEILRSKGDEKIDELVAEISLLRKKAPCSI
SEQ ID NO 4: Nucleotide sequence encoding for SEQ ID NO 3 
ATGGATGCCCAGCGAGGTCACACCACCACCATTTTGATGCTTCCATGGGTCGGCT
ACGGCCATCTCTTGCCTTTCCTCGAGCTGGCCAAAAGCCTCTCCAGGAGGAAATTA
TTCCACATCTACTTCTGTTCAACGTCTGTTAGCCTCGACGCCATTAAACCAAAGCTT
CCTCCTTCTATCTCTTCTGATGATTCCATCCAACTTGTGGAACTTCGTCTCCCTTCT
TCTCCTGAGTTACCTCCTCATCTTCACACAACCAACGGCCTTCCCTCTCACCTCAT
GCCCGCTCTCCACCAAGCCTTCGTCATGGCCGCCCAACACTTTCAGGTCATTTTAC
AAACACTTGCCCCGCATCTCCTCATTTATGACATTCTCCAACCTTGGGCTCCTCAA
GTGGCTTCATCCCTCAACATTCCAGCCATCAACTTCAGTACTACCGGAGCTTCAAT
 
GCTTTCTCGAACGCTTCACCCTACTCACTACCCAAGTTCTAAATTCCCAATCTCAGG
TTTGTTCTTCACAATCACTGGAGAGCCATGTACACCACCGCCGATGGGGCTCTTAC
AGAAGAAGGCCACAAAATTGAAGAAACACTTGCGAATTGCTTGCATACTTCTTGCG
GGGTAGTTTTGGTCAATAGTTTCAGAGAGCTTGAGACGAAATATATCGATTATCTCT
CTGTTCTCTTGAACAAGAAAGTTGTTCCGGTCGGTCCTTTGGTTTACGAACCGAAT
CAAGAAGGGGAAGATGAAGGTTATTCAAGCATCAAAAATTGGCTTGACAAAAAGGA
ACCGTCCTCAACCGTCTTCGTTTCATTTGGAACCGAATACTTCCCGTCAAAGGAAG
AAATGGAAGAGATAGCGTATGGGTTAGAGCTGAGCGAGGTTAATTTCATCTGGGTC
CTTAGATTTCCTCAAGGAGACAGCACCAGCACCATTGAAGACGCCTTGCCGAAGG
GGTTTCTGGAGAGAGCGGGAGAGAGGGCGATGGTGGTGAAGGGTTGGGCTCCTC
AGGCGAAGATACTGAAGCATTGGAGCACAGGGGGGCTTGTGAGTCACTGTGGATG
GAACTCGATGATGGAGGGCATGATGTTTGGCGTACCCATAATAGCGGTCCCGATG
CATCTGGACCAGCCCTTTAACGCCGGACTCTTGGAAGAAGCTGGCGTCGGCGTGG
AAGCCAAGCGAGGTTCGGACGGCAAAATTCAAAGAGAAGAAGTTGCAAAGTCGAT
CAAAGAAGTGGTGATTGAGAAAACCAGGGAAGACGTGAGGAAGAAAGCAAGAGAA
ATGGGTGAGATTTTGAGGAGTAAAGGAGATGAGAAAATTGATGAGTTGGTGGCTG
AAATTTCTCTTTTGCGCAAAAAGGCTCCATGTTCAATTTAA
SEQ ID NO 5: *Arabidopsis thaliana* 
MSPILGYWKIKGLVQPTRLLLEYLEEKYEEHLYERDEGDKWRNKKFELGLEFPNLPYYI
DGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKL
VCFKKRIEAIPQIDKYLKSSKYIAWPLQGWQATFGGGDHPPKSDLVPRGSVSETTKSS
PLHFVLFPFMAQGHMIPMVDIARLLAQRGVIITIVTTPHNAARFKNVLNRAIESGLPINLV
QVKFPYLEAGLQEGQENIDSLDTMERMIPFFKAVNFLEEPVQKLIEEMNPRPSCLISDF
CLPYTSKIAKKFNIPKILFHGMGCFCLLCMHVLRKNREILDNLKSDKELFTVPDFPDRVE
FTRTQVPVETYVPAGDWKDIFDGMVEANETSYGVIVNSFQELEPAYAKDYKEVRSGKA
WTIGPVSLCNKVGADKAERGNKSDIDQDECLKWLDSKKHGSVLYVCLGSICNLPLSQL
KELGLGLEESQRPFIWVIRGWEKYKELVEWFSESGFEDRIQDRGLLIKGWSPQMLILS
HPSVGGFLTHCGWNSTLEGITAGLPLLTWPLFADQFCNEKLVVEVLKAGVRSGVEQP
 
MKWGEEEKIGVLVDKEGVKKAVEELMGESDDAKERRRRAKELGDSAHKAVEEGGSS
HSNISFLLQDIMELAEPNNAAAS
SEQ ID NO 6: Nucleotide sequence encoding for SEQ ID NO 5 
ATGAGGCATGGATCCGTTAGCGAAACCACCAAAAGCAGTCCGCTGCATTTTGTTCT
GTTTCCGTTTATGGCACAGGGTCATATGATTCCGATGGTTGATATTGCACGTCTGC
TGGCACAGCGTGGTGTGATTATTACCATTGTTACCACACCGCATAATGCAGCACGC
TTTAAAAACGTTCTGAATCGTGCAATTGAAAGCGGTCTGCCGATTAATCTGGTTCA
GGTTAAATTTCCGTATCTGGAAGCAGGTCTGCAAGAAGGTCAAGAAAATATTGATA
GCCTGGATACCATGGAACGCATGATTCCGTTTTTCAAAGCCGTGAATTTTCTGGAA
GAACCGGTGCAGAAACTGATCGAAGAAATGAATCCGCGTCCGAGCTGTCTGATTA
GCGATTTTTGTCTGCCGTATACCAGCAAAATCGCCAAAAAATTCAACATCCCGAAA
ATCCTGTTTCATGGTATGGGTTGTTTTTGCCTGCTGTGTATGCATGTTCTGCGTAAA
AATCGTGAAATCCTGGATAACCTGAAAAGCGATAAAGAACTGTTTACCGTTCCGGA
TTTTCCGGATCGTGTGGAATTTACCCGTACACAGGTTCCGGTTGAAACCTATGTTC
CGGCAGGCGATTGGAAAGATATTTTTGATGGTATGGTGGAAGCCAACGAAACCAG
CTATGGTGTTATTGTGAATAGCTTTCAAGAACTGGAACCGGCATATGCGAAAGATT
ACAAAGAAGTTCGTAGCGGTAAAGCATGGACCATTGGTCCGGTTAGCCTGTGT
AATAAAGTTGGTGCAGATAAAGCAGAACGCGGTAATAAAAGTGATATCGATCAGGA
TGAATGCCTGAAATGGCTGGATAGCAAAAAACATGGTAGCGTTCTGTATGTTTGTC
TGGGTAGCATTTGCAATCTGCCGCTGAGCCAGCTGAAAGAATTAGGTCTGGGTTTA
GAAGAAAGCCAGCGTCCGTTTATTTGGGTTATTCGTGGTTGGGAGAAATACAAAGA
ACTGGTTGAATGGTTTAGCGAAAGCGGTTTTGAAGATCGTATTCAGGATCGTGGCC
TGCTGATTAAAGGTTGGAGTCCGCAGATGCTGATTCTGAGCCATCCGAGCGTTGG
TGGCTTTCTGACCCATTGTGGTTGGAATAGCACCCTGGAAGGTATTACAGCTGGCC
TGCCGCTGCTGACCTGGCCTCTGTTTGCAGATCAGTTTTGTAATGAAAAACTGGTG
GTGGAAGTTCTGAAAGCCGGTGTGCGTAGCGGTGTTGAACAGCCGATGAAATGGG
GTGAAGAAGAAAAAATTGGCGTCCTGGTTGATAAAGAAGGTGTTAAAAAAGCCGTG
GAAGAACTGATGGGTGAAAGTGATGATGCAAAAGAACGTCGTCGTCGTGCAAAAG
AGCTGGGCGATAGCGCACATAAAGCAGTTGAAGAAGGTGGTAGCAGCCATAGCAA
 
TATTAGCTTTCTGCTGCAGGATATTATGGAACTGGCAGAACCGAATAACTAAGCGG
CCGCTGAA
SEQ ID NO 7: *Arabidopsis thaliana* 
MAFEKNNEPFPLHFVLFPFMAQGHMIPMVDIARLLAQRGVLITIVTTPHNAARFKNVLN
RAIESGLPINLVQVKFPYQEAGLQEGQENMDLLTTMEQITSFFKAVNLLKEPVQNLIEE
MSPRPSCLISDMCLSYTSEIAKKFKIPKILFHGMGCFCLLCVNVLRKNREILDNLKSDKE
YFIVPYFPDRVEFTRPQVPVETYVPAGWKEILEDMVEADKTSYGVIVNSFQELEPAYAK
DFKEARSGKAWTIGPVSLCNKVGVDKAERGNKSDIDQDECLEWLDSKEPGSVLYVCL
GSICNLPLSQLLELGLGLEESQRPFIWVIRGWEKYKELVEWFSESGFEDRIQDRGLLIK
GWSPQMLILSHPSVGGFLTHCGWNSTLEGITAGLPMLTWPLFADQFCNEKLVVQILKV
GVSAEVKEVMKWGEEEKIGVLVDKEGVKKAVEELMGESDDAKERRRRAKELGESAH
KAVEEGGSSHSNITFLLQDIMQLAQSNN
SEQ ID NO 8: Nucleotide sequence encoding for SEQ ID NO 7 
ATGGCTTTCGAAAAAAACAACGAACCTTTTCCTCTTCACTTTGTTCTCTTCCCTTTCA
TGGCTCAAGGCCACATGATTCCCATGGTTGATATTGCAAGGCTCTTGGCTCAGCGA
GGTGTGCTTATAACAATTGTCACGACGCCTCACAATGCAGCAAGGTTCAAGAATGT
CCTAAACCGTGCCATTGAGTCTGGTTTGCCCATCAACCTAGTGCAAGTCAAGTTTC
CATATCAAGAAGCTGGTCTGCAAGAAGGACAAGAAAATATGGATTTGCTTACCACG
ATGGAGCAGATAACATCTTTCTTTAAAGCGGTTAACTTACTCAAAGAACCAGTCCAG
AACCTTATTGAAGAGATGAGCCCGCGACCAAGCTGTCTAATCTCTGATATGTGTTT
GTCGTATACAAGCGAAATCGCCAAGAAGTTCAAAATACCAAAGATCCTCTTCCATG
GCATGGGTTGCTTTTGTCTTCTGTGTGTTAACGTTCTGCGCAAGAACCGTGAGATC
TTGGACAATTTAAAGTCTGATAAGGAGTACTTCATTGTTCCTTATTTTCCTGATAGA
GTTGAATTCACAAGACCTCAAGTTCCGGTGGAAACATATGTTCCTGCAGGCTGGAA
AGAGATCTTGGAGGATATGGTAGAAGCGGATAAGACATCTTATGGTGTTATAGTCA
ACTCATTTCAAGAGCTCGAACCTGCGTATGCCAAAGACTTCAAGGAGGCAAGGTCT
GGTAAAGCATGGACCATTGGACCTGTTTCCTTGTGCAACAAGGTAGGAGTAGACAA
 
AGCAGAGAGGGGAAACAAATCAGATATTGATCAAGATGAGTGCCTTGAATGGCTC
GATTCTAAGGAACCGGGATCTGTGCTCTACGTTTGCCTTGGAAGTATTTGTAATCTT
CCTCTGTCTCAGCTCCTTGAGCTGGGACTAGGCCTAGAGGAATCCCAAAGACCTTT
CATCTGGGTCATAAGAGGTTGGGAGAAATACAAAGAGTTAGTTGAGTGGTTCTCGG
AAAGCGGCTTTGAAGATAGAATCCAAGATAGAGGACTTCTCATCAAAGGATGGTCC
CCTCAAATGCTTATCCTTTCACATCCTTCTGTTGGAGGGTTCTTAACGCACTGCGG
ATGGAACTCGACTCTTGAGGGGATAACTGCTGGTCTACCAATGCTTACATGGCCAC
TATTTGCAGACCAATTCTGCAACGAGAAACTGGTCGTACAAATACTAAAAGTCGGT
GTAAGTGCCGAGGTTAAAGAGGTCATGAAATGGGGAGAAGAAGAGAAGATAGGAG
TGTTGGTGGATAAAGAAGGAGTGAAGAAGGCAGTGGAAGAACTAATGGGTGAGAG
TGATGATGCAAAAGAGAGAAGAAGAAGAGCCAAAGAGCTTGGAGAATCAGCTCAC
AAGGCTGTGGAAGAAGGAGGCTCCTCTCATTCTAATATCACTTTCTTGCTACAAGA
CATAATGCAACTAGCACAGTCCAATAAT
SEQ ID NO 9: *Siraitia grosvenorii* 
MVQPRVLLFPFPALGHVKPFLSLAELLSDAGIDVVFLSTEYNHRRISNTEALASRFPTLH
FETIPDGLPPNESRALADGPLYFSMREGTKPRFRQLIQSLNDGRWPITCIITDIMLSSPI
EVAEEFGIPVIAFCPCSARYLSIHFFIPKLVEEGQIPYADDDPIGEIQGVPLFEGLLRRN
HLPGSWSDKSADISFSHGLINQTLAAGRASALILNTFDELEAPFLTHLSSIFNKIYTIGP
LHALSKSRLGDSSSSASALSGFWKEDRACMSWLDCQPPRSVVFVSFGSTMKMKADE
LREFWYGLVSSGKPFLCVLRSDVVSGGEAAELIEQMAEEEGAGGKLGMVVEWAAQE
KVLSHPAVGGFLTHCGWNSTVESIAAGVPMMCWPILGDQPSNATWIDRVWKIGVERN
NREWDRLTVEKMVRALMEGQKRVEIQRSMEKLSKLANEKVVRGGLSFDNLEVLVEDI
KKLKPYKF
SEQ ID NO 10: Nucleotide sequence encoding for SEQ ID NO 9 
ATGGTGCAACCTCGGGTACTGCTGTTTCCTTTCCCGGCACTGGGCCACGTGAAGC
CCTTCTTATCACTGGCGGAGCTGCTTTCCGACGCCGGCATAGACGTCGTCTTCCTC
AGCACCGAGTATAACCACCGTCGGATCTCCAACACTGAAGCCCTAGCCTCCCGCT
TCCCGACGCTTCATTTCGAAACTATACCGGATGGCCTGCCGCCTAATGAGTCGCG
 
CGCTCTTGCCGACGGCCCACTGTATTTCTCCATGCGTGAGGGAACTAAACCGAGA
TTCCGGCAACTGATTCAATCTCTTAACGACGGTCGTTGGCCCATCACCTGTATTAT
CACTGACATCATGTTATCTTCTCCGATTGAAGTAGCGGAAGAATTTGGGATTCCAG
TAATTGCCTTCTGCCCCTGCAGTGCTCGCTACTTATCGATTCACTTTTTTATACCGA
AGCTCGTTGAGGAAGGTCAAATTCCATACGCAGATGACGATCCGATTGGAGAGAT
CCAGGGGGTGCCCTTGTTCGAAGGTCTTTTGCGACGGAATCATTTGCCTGGTTCTT
GGTCTGATAAATCTGCAGATATATCTTTCTCGCATGGCTTGATTAATCAGACCCTTG
CAGCTGGTCGAGCCTCGGCTCTTATACTCAACACCTTCGACGAGCTCGAAGCTCC
ATTTCTGACCCATCTCTCTTCCATTTTCAACAAAATCTACACCATTGGACCCCTCCA
TGCTCTGTCCAAATCAAGGCTCGGCGACTCCTCCTCCTCCGCTTCTGCCCTCTCC
GGATTCTGGAAAGAGGATAGAGCCTGCATGTCCTGGCTCGACTGTCAGCCGCCGA
GATCTGTGGTTTTCGTCAGTTTCGGGAGTACGATGAAGATGAAAGCCGATGAATTG
AGAGAGTTCTGGTATGGGTTGGTGAGCAGCGGGAAACCGTTCCTCTGCGTGTTGA
GATCCGACGTTGTTTCCGGCGGAGAAGCGGCGGAATTGATCGAACAGATGGCGG
AGGAGGAGGGAGCTGGAGGGAAGCTGGGAATGGTAGTGGAGTGGGCAGCGCAA
GAGAAGGTCCTGAGCCACCCTGCCGTCGGTGGGTTTTTGACGCACTGCGGGTGG
AACTCAACGGTGGAAAGCATTGCCGCGGGAGTTCCGATGATGTGCTGGCCGATTC
TCGGCGACCAACCCAGCAACGCCACTTGGATCGACAGAGTGTGGAAAATTGGGGT
TGAAAGGAACAATCGTGAATGGGACAGGTTGACGGTGGAGAAGATGGTGAGAGCA
TTGATGGAAGGCCAAAAGAGAGTGGAGATTCAGAGATCAATGGAGAAGCTTTCAAA
GTTGGCAAATGAGAAGGTTGTCAGGGGTGGGTTGTCTTTTGATAACTTGGAAGTTC
TCGTTGAAGACATCAAAAAATTGAAACCATATAAATTTTAA
SEQ ID NO 11: *Siraitia grosvenorii* 
MEKGDTHILVFPFPSQGHINPLLQLSKRLIAKGIKVSLVTTLHVSNHLQLQGAYSNSVKI
EVISDGSEDRLETDTMRQTLDRFRQKMTKNLEDFLQKAMVSSNPPKFILYDSTMPWVL
EVAKEFGLDRAPFYTQSCALNSINYHVLHGQLKLPPETPTISLPSMPLLRPSDLPAYDF
DPASTDTIIDLLTSQYSNIQDANLLFCNTFDKLEGEIIQWMETLGRPVKTVGPTVPSAYL
DKRVENDKHYGLSLFKPNEDVCLKWLDSKPSGSVLYVSYGSLVEMGEEQLKELALGIK
ETGKFFLWVVRDTEAEKLPPNFVESVAEKGLVVSWCSQLEVLAHPSVGCFFTHCGWN
 
STLEALCLGVPVVAFPQWADQVTNAKFLEDVWKVGKRVKRNEQRLASKEEVRSCIWE
VMEGERASEFKSNSMEWKKWAKEAVDEGGSSDKNIEEFVAMLKQT
SEQ ID NO 12: Nucleotide sequence encoding for SEQ ID NO 11 
ATGGAGAAAGGCGATACGCATATTCTAGTGTTTCCTTTCCCTTCACAAGGCCACAT
AAACCCTCTTCTTCAACTATCGAAGCGCCTAATCGCCAAGGGAATCAAGGTTTCGC
TGGTCACAACCTTACATGTTAGCAATCACTTGCAGTTGCAGGGTGCTTATTCCAACT
CCGTGAAGATCGAAGTCATTTCCGATGGCTCTGAGGATCGTCTGGAAACCGATACT
ATGCGCCAAACTCTGGATCGATTTCGGCAGAAGATGACGAAGAACTTGGAAGATTT
CTTGCAGAAAGCCATGGTTTCTTCAAATCCGCCTAAATTCATTCTGTATGATTCGAC
AATGCCGTGGGTTTTGGAGGTCGCCAAGGAGTTCGGACTCGATAGGGCCCCGTTC
TACACTCAGTCTTGTGCGCTTAACAGTATCAATTATCATGTTCTTCATGGTCAATTG
AAGCTTCCTCCTGAAACCCCCACGATTTCGTTGCCTTCTATGCCTCTGCTTCGCCC
CAGCGATCTCCCGGCTTATGATTTTGATCCTGCCTCCACTGACACCATCATCGATC
TTCTTACCAGTCAGTATTCTAATATCCAGGATGCAAATCTGCTTTTCTGCAACACTTT
TGACAAGTTGGAAGGCGAGATTATCCAATGGATGGAGACCCTGGGTCGCCCTGTG
AAAACCGTAGGACCAACTGTTCCATCAGCCTACTTAGACAAAAGGGTAGAGAACGA
CAAGCACTATGGGCTGAGTCTGTTCAAGCCCAACGAGGACGTCTGCCTCAAATGG
CTTGATAGCAAGCCCTCTGGTTCTGTTCTGTATGTGTCTTATGGCAGTTTGGTTGAA
ATGGGGGAAGAGCAGCTGAAGGAGTTGGCTCTGGGAATCAAGGAAACTGGCAAG
TTCTTCTTGTGGGTGGTGAGAGACACTGAAGCAGAGAAGCTTCCTCCCAACTTTGT
GGAGAGTGTGGCAGAGAAGGGGCTTGTGGTCAGCTGGTGCTCCCAGCTGGAGGT
ATTGGCTCACCCCTCCGTCGGCTGCTTCTTCACGCACTGTGGCTGGAACTCGACG
CTTGAGGCGCTGTGCTTGGGCGTCCCGGTGGTCGCTTTCCCACAGTGGGCTGATC
AGGTAACCAATGCAAAGTTTTTGGAAGATGTTTGGAAGGTTGGGAAGAGGGTGAA
GCGGAATGAGCAGAGGCTGGCAAGTAAAGAAGAAGTAAGGAGTTGCATTTGGGAA
GTGATGGAGGGAGAGAGAGCCAGCGAGTTCAAGAGCAACTCCATGGAGTGGAAG
AAGTGGGCAAAAGAAGCTGTGGATGAAGGTGGGAGCTCTGATAAGAACATTGAGG
AGTTTGTGGCCATGCTCAAGCAAACTTGA
SEQ ID NO 13: *Barbarea vulgaris* 
MVSEITHKSYPLHFVLFPFMAQGHMIPMVDIARLLAQRGVKITIVTTPHNAARFENVLSR
AIESGLPISIVQVKLPSQEAGLPEGNETFDSLVSMELLVPFFKAVNMLEEPVQKLFEEM
SPQPSCIISDFCLPYTSKIAKKFNIPKILFHGMCCFCLLCMHVLRKNREILENLKSDKEHF
VVPYFPDRVEFTRPQVPMATYVPGEWHEIKEDIVEADKTSYGVIVNTYQELEPAYAND
YKEARSGKAWTIGPVSLCNKVGADKAERGNKADIDQDECLKWLDSKEEGSVLYVCLG
SICSLPLSQLKELGLGLEESQRPFIWVVRGWEKNKELLEWFSESGFEERVKDRGLLIK
GWSPQMLILAHHSVGGFLTHCGWNSTLEGITSGIPLLTWPLFGDQFCNQKLVVQVLKV
GVSAGVEEVTNWGEEEKIGVLVDKEGVKKAVEELMGESDDAKERRKRVKELGQLAQK
AVEEGGSSHSNITSLLEDIMQLAQSNN.
SEQ ID NO 14: Nucleotide sequence encoding for SEQ ID NO 13 
ATGGTTTCCGAAATCACCCATAAATCTTATCCTCTTCACTTTGTTCTCTTCCCTTTCA
TGGCTCAAGGCCACATGATTCCCATGGTTGATATTGCAAGGCTCTTGGCTCAGCGC
GGTGTGAAAATAACAATTGTCACAACGCCGCACAATGCAGCGAGGTTCGAGAATG
TCCTAAGCCGTGCCATTGAGTCTGGCTTGCCCATCAGCATAGTGCAAGTCAAGCTT
CCATCTCAAGAAGCTGGCTTACCAGAAGGAAATGAGACTTTCGATTCACTTGTCTC
GATGGAGTTGCTGGTACCTTTCTTTAAAGCGGTTAACATGCTTGAAGAACCGGTCC
AGAAGCTCTTTGAAGAGATGAGCCCTCAACCAAGCTGTATAATTTCTGATTTTTGTT
TGCCTTATACAAGCAAAATAGCCAAGAAGTTCAATATCCCAAAGATCCTCTTCCATG
GCATGTGTTGCTTTTGTCTTCTGTGTATGCATGTTTTACGCAAAAACCGTGAGATCT
TGGAAAACTTAAAGTCTGACAAGGAGCATTTCGTTGTTCCTTATTTTCCTGATCGAG
TTGAATTCACAAGACCTCAAGTTCCAATGGCAACATATGTTCCTGGAGAGTGGCAC
GAGATCAAGGAGGATATAGTAGAAGCGGATAAGACTTCCTATGGTGTGATAGTCAA
CACATATCAAGAGCTCGAGCCTGCTTATGCCAACGACTACAAGGAGGCAAGGTCT
GGTAAAGCATGGACCATTGGACCTGTTTCCTTGTGCAACAAGGTGGGAGCCGACA
AAGCAGAGAGGGGAAACAAAGCAGACATTGATCAAGATGAGTGTCTTAAATGGCTT
GATTCTAAAGAAGAAGGTTCGGTTCTATATGTTTGCCTTGGAAGTATCTGCAGTCTT
CCTCTGTCTCAGCTCAAAGAGCTGGGGCTAGGCCTTGAGGAATCCCAAAGACCTT
TCATTTGGGTCGTAAGAGGTTGGGAGAAGAACAAAGAGTTACTTGAGTGGTTCTCG
 
GAGAGCGGATTTGAAGAAAGAGTAAAAGACAGAGGGCTTCTCATCAAAGGATGGT
CACCTCAAATGCTTATCCTTGCACATCATTCCGTTGGAGGGTTCTTAACACACTGTG
GATGGAACTCGACCCTCGAAGGAATCACTTCAGGCATTCCATTGCTCACTTGGCCA
CTGTTTGGAGACCAATTCTGCAACCAAAAACTTGTCGTGCAGGTGCTAAAAGTGGG
TGTAAGTGCCGGGGTTGAAGAGGTTACGAATTGGGGAGAAGAGGAGAAAATAGGA
GTATTAGTGGATAAAGAGGGAGTGAAGAAGGCAGTTGAAGAATTAATGGGTGAGA
GTGATGATGCTAAAGAAAGAAGAAAAAGAGTCAAAGAGCTTGGACAATTAGCTCAA
AAGGCTGTGGAGGAAGGAGGCTCATCTCATTCTAATATCACATCCTTGCTAGAAGA
CATAATGCAACTAGCACAATCTAATAATTGA
SEQ ID NO 15: *Barbarea vulgaris* 
MVSEITHKSYPLHFVLFPFMAQGHMIPMVDIARLLAQRGVKITIVTTPHNAARFENVLSR
AIESGLPISIVQVKLPSQEAGLPEGNETFDSLVSTKLLVPFFKAVNMLEEPVQKLFEEMS
PQPSCIISDFCLPYTSKIAKKFNIPKILFHGMCCFCLLCMHVLRKNREILENLKSDKEHFV
VPYFPDRVEFTRPQVPLATYVPGEWHEIKEDMVEADKTSYGVIVNTYQELEPAYANGY
KEARSGKAWTIGPVSLCNKVGADKAERGNKADIDQDECLKWLDSKEEGSVLYVCLGSI
CSLPLSQLKELGLGLEESQRPFIWVVRGWEKNKELLEWFSESGFEERVKDRGLLIKG
WSPQMLILAHHSVGGFLTHCGWNSTLEGITSGVPLLTWPLFGDQFCNQKLVVQVLKV
GVSAGVEEVTNWGEEEKIGVLVDKEGVKKAVEELMGESDDAKEIRKRVKELGQLAHK
AVEEGGSSHSNITSLLEDIMQLAQPNN
SEQ ID NO 16: Nucleotide sequence encoding for SEQ ID NO 15 
ATGGTTTCCGAAATCACCCATAAATCTTATCCTCTTCACTTTGTTCTCTTCCCTTTCA
TGGCTCAAGGCCACATGATTCCCATGGTTGATATTGCAAGGCTCTTGGCTCAGCGC
GGTGTGAAAATAACAATTGTCACAACGCCGCACAATGCAGCGAGGTTCGAGAATG
TCCTAAGCCGTGCCATTGAGTCTGGCTTGCCCATCAGCATAGTGCAAGTCAAGCTT
CCATCTCAAGAAGCTGGCTTACCAGAAGGAAATGAGACTTTCGATTCACTTGTCTC
AACAAAGTTGCTGGTACCTTTCTTTAAAGCGGTTAACATGCTTGAAGAACCGGTCC
AGAAGCTCTTTGAAGAGATGAGCCCTCAACCAAGCTGTATAATTTCTGATTTTTGTT
TGCCTTATACAAGCAAAATCGCCAAGAAGTTCAATATCCCAAAGATCCTCTTCCATG
 
GCATGTGTTGCTTTTGTCTTCTGTGTATGCATGTTTTACGCAAGAACCGTGAGATCT
TGGAAAACTTAAAGTCTGACAAGGAGCATTTCGTTGTTCCTTATTTTCCTGATCGAG
TTGAATTCACAAGACCTCAAGTTCCATTGGCAACATATGTTCCTGGGGAATGGCAC
GAGATCAAGGAGGATATGGTAGAAGCGGATAAGACTTCCTATGGTGTGATAGTCAA
CACATATCAAGAGCTCGAGCCTGCTTATGCCAACGGCTACAAGGAGGCAAGGTCT
GGTAAAGCATGGACCATTGGACCTGTTTCCTTGTGCAACAAGGTGGGAGCCGACA
AAGCAGAGAGGGGAAACAAAGCAGACATTGATCAAGATGAGTGTCTTAAATGGCTT
GATTCTAAAGAAGAAGGTTCGGTTCTATATGTTTGCCTTGGAAGTATCTGCAGTCTT
CCTCTGTCTCAGCTCAAGGAGCTGGGGCTAGGCCTTGAGGAATCCCAAAGACCTT
TCATTTGGGTCGTAAGAGGTTGGGAGAAGAACAAAGAGTTACTTGAGTGGTTCTCG
GAGAGCGGATTTGAAGAAAGAGTAAAAGACAGAGGGCTTCTCATCAAAGGATGGT
CACCTCAAATGCTTATCCTTGCACATCATTCCGTTGGAGGGTTCTTAACACACTGTG
GATGGAACTCGACCCTCGAAGGAATCACTTCAGGCGTTCCATTGCTCACTTGGCC
ACTGTTTGGAGACCAATTCTGCAACCAAAAACTTGTCGTGCAGGTGCTAAAAGTGG
GTGTAAGTGCCGGGGTTGAAGAGGTTACGAATTGGGGAGAAGAGGAGAAAATAGG
AGTATTAGTGGATAAAGAGGGAGTGAAGAAGGCAGTGGAAGAATTAATGGGTGAG
AGTGATGATGCTAAAGAAATAAGAAAAAGAGTCAAAGAGCTTGGACAATTAGCTCA
CAAGGCTGTGGAGGAAGGAGGCTCATCTCATTCTAATATCACATCCTTGCTAGAAG
ACATAATGCAACTAGCACAACCTAATAATTGA
SEQ ID NO 17: *Barbarea vulgaris* 
MASETTHQFHSPLHFVLFPFMAQGHMIPMVDIARILAQRGVTITIVTTPHNAARFKNVLS
RAIQSGLPINLVQAKFPSQESGSLEGHENLDLLDSLGASLTFFKATNMFEKPVEKLLKEI
QPRPSCIIGDMCLPYTNRIAKNLGIPKIIFHGMCCFNLLCMHIMRQNYEFLETIDSEKEYF
PIPNFPERAEFTKSQLPMITYAGEFKEFLDEVTEGDNTSYGVIVNTFEELEPAYVRDYK
KVKAGKVWSIGPVSLCNKVGEDKAERGNKAAIDQDECIKWLDSKEEGSVLYVCLGSIC
NLPLSQLKELGLGLEESQRPFIWVIRGWEKYNELAEWISESGFKERIKERGLLIRGWSP
QMLILSHPAVGGFLTHCGWNSTLEGITSGVPLLTWPLFGDQFCNEKLVVQVLKVGVRS
GVEESMKWGQEENIGVLVDKEGVKKAVEEVMGESDDAKERRRRVKELGQLAHKAVE
EGGSSHSNITSLLQDIRQLQSKK
SEQ ID NO 18: Nucleotide sequence encoding for SEQ ID NO 17 
ATGGCTTCTGAAACTACACATCAATTCCATTCACCATTACATTTCGTTTTGTTCCCTT
TTATGGCACAAGGTCATATGATCCCAATGGTTGATATCGCTAGAATCTTGGCACAA
AGAGGTGTTACTATCACAATCGTTACTACACCACATAACGCTGCAAGATTCAAAAAC
GTTTTGTCTAGAGCTATCCAATCAGGTTTGCCAATTAATTTGGTTCAAGCAAAGTTC
CCATCTCAAGAATCTGGTTCATTGGAAGGTCATGAAAATTTGGATTTGTTGGATTCT
TTAGGTGCTTCATTGACTTTCTTTAAGGCAACAAACATGTTCGAAAAGCCAGTTGAA
AAGTTGTTAAAAGAAATTCAACCAAGACCATCATGTATTATTGGTGACATGTGTTTG
CCATACACTAACAGAATTGCTAAAAATTTGGGTATTCCAAAAATTATTTTTCATGGCA
TGTGTTGTTTTAATTTGTTATGTATGCATATTATGAGACAAAACTACGAATTCTTGGA
AACAATCGATTCTGAAAAGGAATACTTCCCAATCCCAAACTTCCCAGAAAGAGCTG
AATTCACTAAGTCACAATTGCCAATGATCACATACGCTGGTGAATTCAAAGAATTCT
TGGATGAAGTTACTGAAGGTGACAACACATCTTACGGTGTTATTGTTAACACTTTCG
AAGAATTAGAACCAGCTTACGTTAGAGATTACAAGAAAGTTAAGGCTGGTAAAGTTT
GGTCTATTGGTCCAGTTTCATTGTGTAATAAGGTTGGTGAAGATAAAGCTGAAAGA
GGTAATAAGGCTGCAATCGATCAAGATGAATGTATCAAGTGGTTGGATTCTAAGGA
AGAAGGTTCAGTTTTGTACGTTTGTTTGGGTTCTATCTGTAATTTGCCATTGTCACA
ATTGAAAGAATTGGGTTTAGGTTTGGAAGAATCTCAAAGACCTTTTATTTGGGTTAT
TAGAGGTTGGGAAAAGTACAACGAATTAGCTGAATGGATCTCTGAATCAGGTTTTA
AAGAAAGAATTAAAGAAAGAGGTTTGTTAATTAGAGGTTGGTCTCCACAAATGTTAA
TTTTGTCACATCCAGCAGTTGGTGGTTTCTTGACTCATTGTGGTTGGAATTCTACTT
TGGAAGGTATTACATCAGGTGTTCCATTGTTAACATGGCCATTATTTGGTGACCAAT
TCTGTAACGAAAAGTTGGTTGTTCAAGTTTTGAAAGTTGGTGTTAGATCTGGTGTTG
AAGAATCAATGAAATGGGGTCAAGAAGAAAACATCGGTGTTTTGGTTGATAAAGAA
GGTGTTAAGAAAGCTGTTGAAGAAGTTATGGGTGAATCTGATGATGCAAAGGAAAG
AAGAAGAAGAGTTAAGGAATTAGGTCAATTGGCTCATAAAGCAGTTGAAGAAGGTG
GTTCTTCACATTCTAACATCACATCATTGTTGCAAGATATTAGACAATTGCAATCTAA
GAAATAA
SEQ ID NO 19: *Barbarea vulgaris* 
MASAKLHQFHPSLHFVLFPFMAQGHMIPMIDIARLLAQRGVTITIVTTLHNSARFRNVLS
RAIESGLPINLVHVKFPYQEAGLPEGQENIDSLDSKELTVPFFKAVNMLEEPVMKLMEE
MKPRPSCLISDLCLPYTSKIAKKFNIPKIVFHGMGCFCLLCMHVLRRNLEILQNLKSDKE
YFWIPNFPDRVEFTKPQVPVRINASGDWKVFLDEMVKAEETSYGVIVNTFQELEPAYV
KDFQEARAGKVWSIGPVSLCNKIEADKAERGNKAAIDQDECLKWLDSKEGGSVLYVCL
GSICNLPLVQLIELGLGLEESQRPFIWVIRGWEKYNELSEWISESGFEERVKDRGLLIRG
WAPQVLILSHPSVGGFLTHCGWNSTLEGITSGVPLLTLPLFGDQFCNQKLIVQVLKVGV
SAGVEEVMGWGEEEKIGVLVDKERVKKTVEEVMGESDDAKERRRRVKELGKLAHKA
VEEGGSSHSNITLFLQDMCQLQSVGIY
SEQ ID NO 20: Nucleotide sequence encoding for SEQ ID NO 19 
ATGGCTTCTGCAAAATTGCATCAATTCCATCCATCATTGCATTTCGTTTTGTTCCCTT
TTATGGCTCAAGGTCATATGATCCCAATGATCGATATCGCTAGATTGTTGGCACAA
AGAGGTGTTACTATCACAATCGTTACTACATTGCATAACTCTGCTAGATTCAGAAAC
GTTTTGTCTAGAGCAATCGAATCAGGTTTGCCAATTAATTTGGTTCATGTTAAGTTC
CCATATCAAGAAGCTGGTTTACCAGAAGGTCAAGAAAACATCGATTCTTTGGATTC
AAAGGAATTAACTGTTCCATTTTTCAAGGCAGTTAACATGTTGGAAGAACCAGTTAT
GAAGTTGATGGAAGAAATGAAGCCAAGACCATCTTGTTTGATCTCAGATTTGTGTTT
GCCATATACATCTAAAATTGCTAAAAAGTTTAATATCCCAAAGATCGTTTTCCATGG
TATGGGTTGTTTTTGTTTGTTATGTATGCATGTTTTAAGAAGAAATTTGGAAATTTTG
CAAAATTTGAAGTCAGATAAGGAATACTTCTGGATCCCAAACTTCCCAGATAGAGTT
GAATTCACTAAACCACAAGTTCCAGTTAGAATTAATGCTTCTGGTGACTGGAAAGTT
TTCTTGGATGAAATGGTTAAAGCAGAAGAAACTTCATATGGTGTTATTGTTAACACA
TTCCAAGAATTAGAACCAGCATACGTTAAAGATTTTCAAGAAGCTAGAGCTGGTAAA
GTTTGGTCTATTGGTCCAGTTTCATTGTGTAATAAGATCGAAGCTGATAAAGCAGAA
AGAGGTAATAAGGCTGCAATCGATCAAGATGAATGTTTGAAGTGGTTGGATTCTAA
AGAAGGTGGTTCAGTTTTGTACGTTTGTTTGGGTTCTATCTGTAATTTGCCATTGGT
TCAATTGATTGAATTGGGTTTAGGTTTGGAAGAATCTCAAAGACCTTTTATTTGGGT
 
TATTAGAGGTTGGGAAAAGTACAACGAATTATCAGAATGGATCTCTGAATCAGGTTT
CGAAGAAAGAGTTAAGGATAGAGGTTTGTTGATCAGAGGTTGGGCTCCACAAGTT
TTAATTTTGTCTCATCCATCAGTTGGTGGTTTCTTGACTCATTGTGGTTGGAATTCTA
CTTTAGAAGGTATTACATCAGGTGTTCCATTGTTAACATTACCATTGTTTGGTGACC
AATTTTGTAACCAAAAGTTGATCGTTCAAGTTTTGAAAGTTGGTGTTTCTGCTGGTG
TTGAAGAAGTTATGGGTTGGGGTGAAGAAGAAAAGATTGGTGTTTTGGTTGATAAG
GAAAGAGTTAAGAAAACTGTCGAAGAAGTTATGGGTGAATCAGATGATGCAAAGGA
AAGAAGAAGAAGAGTTAAGGAATTGGGTAAATTGGCTCATAAAGCAGTTGAAGAAG
GTGGTTCTTCACATTCTAACATCACATTATTTTTGCAAGATATGTGTCAATTACAATC
AGTTGGTATCTATTAA
SEQ ID NO 21: *Sesamum indicum* 
MDTRKRSIRILMFPWLAHGHISAFLELAKSLAKRNFVIYICSSQVNLNSISKNMSSKDSIS
VKLVELHIPTTILPPPYHTTNGLPPHLMSTLKRALDSARPAFSTLLQTLKPDLVLYDFLQS
WASEEAESQNIPAMVFLSTGAAAISFIMYHWFETRPEEYPFPAIYFREHEYDNFCRFKS
SDSGTSDQLRVSDCVKRSHDLVLIKTFRELEGQYVDFLSDLTRKRFVPVGPLVQEVGC
DMENEGNDIIEWLDGKDRRSTVFSSFGSEYFLSANEIEEIAYGLELSGLNFIWVVRFPH
GDEKIKIEEKLPEGFLERVEGRGLVVEGWAQQRRILSHPSVGGFLSHCGWSSVMEGV
YSGVPIIAVPMHLDQPFNARLVEAVGFGEEVVRSRQGNLDRGEVARVVKKLVMGKSG
EGLRRRVEELSEKMREKGEEEIDSLVEELVTVVRRRERSNLKSENSMKKLNVMDDGE
SEQ ID NO 22: Nucleotide sequence encoding for SEQ ID NO 21 
ATGGATACAAGAAAGAGAAGCATCAGGATTCTAATGTTCCCATGGCTTGCTCATGG
CCATATCTCAGCATTCCTCGAGCTGGCGAAGTCACTTGCCAAAAGAAACTTCGTCA
TTTACATTTGTTCTTCACAAGTAAATCTAAATTCCATCAGCAAGAACATGTCATCAAA
AGACTCCATTTCCGTAAAACTTGTTGAGCTTCACATTCCCACCACCATACTTCCCCC
TCCTTACCACACCACCAATGGCCTCCCACCCCACCTCATGTCCACCCTCAAGAGA
GCCCTCGACAGTGCCCGGCCCGCCTTCTCCACCCTCCTCCAAACCCTCAAGCCCG
ACTTGGTTTTATACGATTTCCTCCAGTCGTGGGCCTCGGAGGAGGCCGAGTCGCA
GAATATACCAGCCATGGTGTTTCTGAGTACCGGAGCTGCAGCGATTTCTTTTATTAT
 
GTACCATTGGTTTGAGACCAGACCGGAGGAGTACCCTTTTCCGGCTATATACTTCC
GGGAACACGAGTATGATAACTTCTGCCGTTTTAAGTCTTCCGACAGCGGTACTAGT
GATCAATTGAGAGTCAGCGATTGCGTTAAACGGTCGCACGATTTGGTTCTGATCAA
GACATTCCGTGAACTGGAAGGACAATACGTAGATTTTCTCTCCGACTTGACTCGGA
AGAGATTCGTACCAGTTGGCCCCCTTGTTCAGGAGGTAGGTTGTGATATGGAGAAT
GAAGGAAATGACATCATCGAATGGCTCGACGGGAAAGACCGTCGTTCGACGGTTT
TCTCCTCATTCGGGAGCGAGTACTTCTTGTCTGCCAATGAGATCGAAGAGATAGCT
TATGGGCTGGAGCTAAGCGGGCTTAACTTCATCTGGGTTGTTAGGTTTCCTCATGG
CGACGAGAAAATCAAGATTGAGGAGAAACTGCCGGAAGGGTTTCTTGAGAGAGTG
GAAGGAAGAGGGTTGGTGGTGGAGGGATGGGCACAGCAGAGGAGAATATTGTCA
CATCCGAGTGTTGGAGGGTTTTTGAGCCACTGTGGGTGGAGTTCTGTGATGGAAG
GGGTGTATTCCGGTGTGCCGATTATTGCCGTGCCGATGCATCTTGACCAGCCGTT
CAATGCTAGGTTGGTGGAGGCGGTGGGGTTTGGGGAGGAGGTGGTGAGGAGTAG
ACAAGGAAATCTTGACAGAGGAGAGGTGGCGAGGGTGGTGAAGAAGCTGGTTATG
GGGAAAAGTGGGGAGGGGTTACGGCGGAGGGTGGAGGAGTTGAGTGAGAAGAT
GAGAGAGAAAGGGGAGGAGGAGATTGATTCACTGGTGGAGGAATTGGTGACGGT
GGTTAGGAGGAGAGAGAGATCGAATCTCAAGTCTGAGAATTCTATGAAGAAATTGA
ATGTGATGGATGATGGAGAATAG
SEQ ID NO 23: *Siraitia grosvenorii* 
MDAAQQGDTTTILMLPWLGYGHLSAFLELAKSLSRRNFHIYFCSTSVNLDAIKPKLPSS
FSDSIQFVELHLPSSPEFPPHLHTTNGLPPTLMPALHQAFSMAAQHFESILQTLAPHLLI
YDSLQPWAPRVASSLKIPAINFNTTGVFVISQGXHPIHYPHSKFPFSEFVLHNHWKAMY
STADGASTERTRKRGEAFLYCLHASCSVILINSFRELEGKYMDYLSVLLNKKVVPVGPL
VYEPNQDGEDEGYSSIKNWLDKKEPSSTVFVSFGSEYFPSKEEMEEIAHGLEASEVNF
IWVVRFPQGDNTSGIEDALPKGFLERAGERGMVVKGWAPQAKILKHWSTGGFVSHCG
WNSVMESMMFGVPIIGVPMHVDQPFNAGLVEEAGVGVEAKRDPDGKIQRDEVAK
SEQ ID NO 24: Nucleotide sequence encoding for SEQ ID NO 23 
ATGGATGCTGCCCAACAAGGTGACACCACAACCATTTTGATGCTTCCATGGCTCGG
CTATGGCCATCTTTCAGCTTTTCTCGAGCTGGCCAAAAGCCTCTCAAGGAGGAACT
TCCATATCTACTTCTGTTCAACCTCTGTTAATCTTGACGCCATTAAACCAAAGCTTC
CTTCTTCTTTCTCTGATTCCATTCAATTTGTGGAGCTCCATCTCCCTTCTTCTCCTGA
GTTCCCTCCTCATCTTCACACAACCAACGGCCTTCCCCCTACCCTCATGCCCGCTC
TCCACCAAGCCTTCTCCATGGCTGCCCAGCACTTTGAGTCCATTTTACAAACACTT
GCCCCGCACCTTCTCATTTATGACTCTCTTCAACCTTGGGCTCCTCGGGTAGCTTC
ATCCCTCAAAATTCCGGCCATCAACTTCAATACCACGGGAGTTTTCGTCATTTCTCA
AGGGYTTCACCCTATTCACTACCCACATTCTAAATTCCCATTCTCAGAGTTCGTTCT
TCACAATCATTGGAAAGCCATGTACTCCACTGCCGATGGAGCTTCTACCGAAAGAA
CCCGCAAACGTGGAGAAGCGTTTCTGTATTGCTTGCATGCTTCTTGTAGTGTAATT
CTAATCAATAGTTTCAGAGAGCTCGAGGGGAAATATATGGATTATCTCTCTGTTCTC
TTGAACAAGAAAGTTGTTCCGGTTGGTCCTTTGGTTTACGAACCGAATCAAGACGG
GGAAGATGAAGGTTATTCAAGCATCAAAAATTGGCTTGACAAAAAGGAACCGTCCT
CCACCGTCTTCGTGTCATTTGGAAGCGAATACTTCCCGTCAAAGGAAGAAATGGAA
GAGATAGCCCATGGGTTAGAGGCGAGCGAGGTTAATTTCATCTGGGTCGTTAGG
TTTCCTCAAGGAGACAACACCAGCGGCATTGAAGATGCCTTGCCGAAGGGTTTTCT
GGAGAGGGCGGGAGAGAGAGGGATGGTGGTGAAGGGTTGGGCTCCTCAGGCGA
AGATACTGAAGCATTGGAGCACAGGGGGATTCGTGAGCCACTGTGGATGGAACTC
GGTGATGGAGAGCATGATGTTTGGCGTTCCCATAATAGGGGTTCCGATGCATGTG
GACCAGCCCTTTAACGCCGGACTCGTGGAAGAAGCTGGCGTCGGCGTGGAGGCC
AAGCGAGATCCAGACGGCAAAATTCAAAGAGACGAAGTTGCAAAGTTGATCAAAGA
AGTGGTGGTTGAGAAAACCAGAGAAGATGTGCGGAAGAAAGCAAGAGAAATGAGT
GAGATTTTGAGGAGCAAGGGAGAGGAGAAGTTTGATGAGATGGTCGCTGAAATTT
CTCTCTTGCTTAAAATATGA
SEQ ID NO 25: *Brettanomyces bruxellensis* 
MKFILLSTILSGLLSFAAAAPIILKKRDWDYTKDKIHGVNIGGWLVLEPYITPSLFETFGDD
APVDEYHYTQQLGKEEASKRLQEHWANWITEKDFEYIANLGLNMVRIPIGYWAFELQD
NDPYVQGQQAYLDKALGWAEKNGLKVWIDLHGVPGSQNGFDNSGLRDQIDWQTGS
 
GNVDFTLNVLKEMIEKYSDSDYSDTIIGIELLNEPLGSSLNMDELKVFYSSGYELVRDQD
ASVPVIIQDAFQSDYYWDDFLNTEQDTNIYGVVVDHHHYQVFSTGELQRDMDTRIQTA
CNWGKQEGGEYHWNVCGEFSAALTDCAKWLNGLGRGARYDSSYGGGAYVGSCDD
LYTYDEDYWNNSDVIXQYRQYVEAQMDAFENGKMGGWVFWCWKTESAVEWDFQRL
AGLNVIPQPLDDRLYPNQCSF
SEQ ID NO 26: Nucleotide sequence encoding for SEQ ID NO 25

ATGAAGTTTATTTTATTGTCCACAATTTTGAGTGGTCTTCTCTCGTTTGCTGCTGCT
GCTCCAATCATTTTAAAGAAACGTGATTGGGATTACACAAAAGATAAAATTCATGGT
GTGAATATTGGAGGTTGGCTTGTTCTAGAGCCATACATTACACCTTCGCTATTTGAG
ACGTTTGGTGACGATGCACCAGTTGATGAATATCACTACACTCAACAGCTAGGCAA
GGAAGAGGCTTCGAAGAGATTACAAGAGCACTGGGCTAACTGGATTACAGAGAAG
GACTTTGAATATATTGCCAATCTGGGTCTTAACATGGTTAGAATTCCAATTGGATAC
TGGGCTTTTGAATTGCAGGACAATGATCCATACGTCCAGGGACAGCAAGCGTATYT
GGACAAGGCTCTTGGCTGGGCAGAAAAAAATGGCTTGAAAGTTTGGATCGATTTG
CATGGTGTTCCAGGCTCTCAAAATGGATTTGATAATTCTGGTTTGCGTGATCAAATC
GACTGGCAAACTGGTTCCGGAAATGTCGATTTCACATTAAACGTGTTGAAAGAAAT
GATTGAGAAATATTCCGATTCCGATTACAGCGACACAATTATTGGAATTGAGTTGTT
GAACGAGCCTTTAGGATCCTCCTTGAACATGGATGAACTTAAGGTCTTCTACAGTA
GTGGCTATGAACTTGTTAGAGACCAGGATGCTTCCGTTCCAGTCATTATTCAAGAT
GCTTTCCAGTCCGATTACTACTGGGATGACTTTTTAAATACTGAGCAAGACACAAAC
ATTTACGGCGTTGTTGTTGATCATCATCACTACCAGGTCTTTTCTACTGGTGAACTT
CAGAGAGACATGGATACACGCATTCAAACCGCATGTAACTGGGGTAAGCAGGAAG
GTGGGGAGTACCACTGGAATGTCTGCGGAGAGTTCTCAGCAGCCCTTACGGATTG
TGCCAAGTGGCTCAATGGTTTAGGAAGGGGTGCCAGATATGACTCTTCATATGGA
GGTGGTGCTTATGTTGGAAGCTGTGATGATTTGTACACGTACGATGAAGACTACTG
GAACAATAGTGATGTCATCAMGCAATACAGGCAGTATGTGGAGGCCCAAATGGAT
GCATTTGAGAATGGAAAGATGGGAGGATGGGTTTTCTGGTGTTGGAAGACAGAAT
CCGCTGTTGAGTGGGACTTCCAGAGGTTGGCTGGCCTTAACGTCATTCCTCAGCC
TTTGGATGACAGGTTATATCCTAACCAGTGTAGCTTCTAA.

The present invention is also described by the following embodiments:
1. A method for preparing siamenoside I having the structure of formula (I) 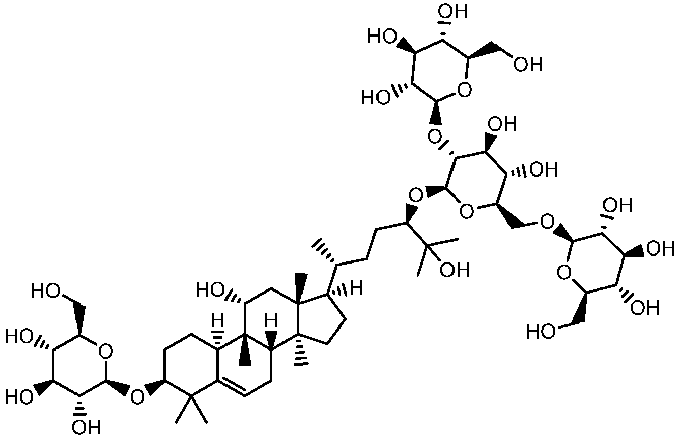 comprising contacting a mogroside having a structure of formula (II) 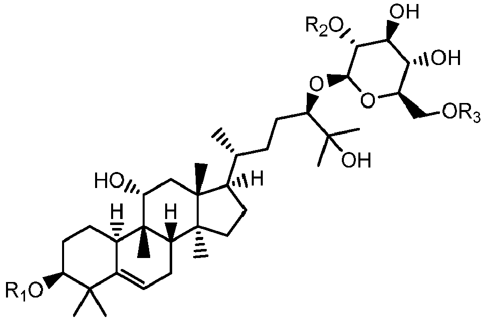 with a polypeptide having UDP-glycotransferase activity.
2. The method of embodiment 1 wherein the step of contacting a mogroside having a structure of formula (II) with a polypeptide having UDP-glycotransferase activity comprises contacting said mogroside having a structure of formula (II) with a recombinant host cell that comprises a gene encoding the polypeptide having UDP-glycotransferase activity.
3. The method of embodiment 1 or 2 wherein the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21 or SEQ ID NO: 23.
4. The method of any of the previous embodiments wherein the mogroside is Mog III and has a structure of formula (III) 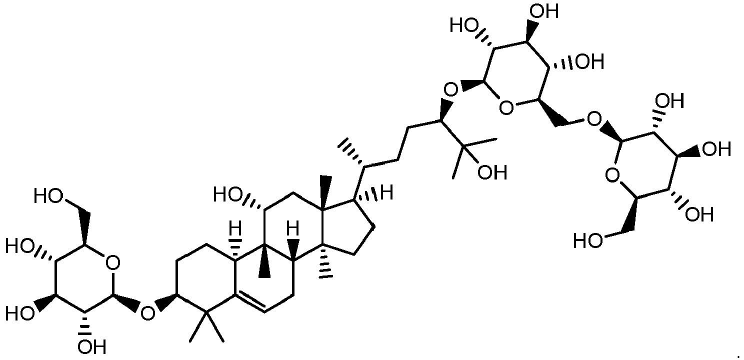
5. The method of embodiment 4 wherein where the method requires contacting mogroside III, the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 1 or SEQ ID NO: 3.
6. The method of any of embodiments 1 to 3 wherein the mogroside is Mog IIIA and has a structure of formula (IV) 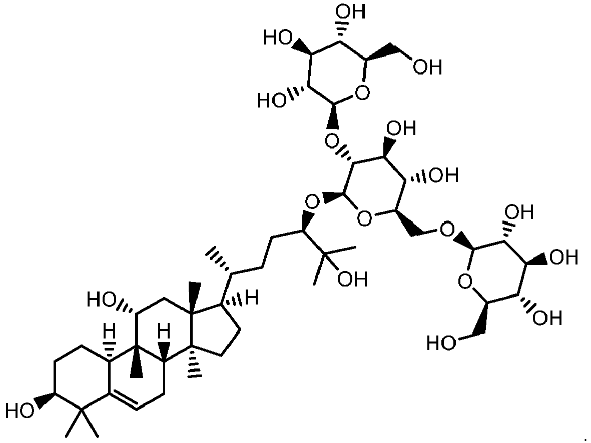
7. The method of embodiment 6 wherein where the method requires contacting mogroside IIIA the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 5, SEQ ID NO: 7, or SEQ ID NO: 9, SEQ ID NO: 11, SEQ ID NO: 13, SEQ ID NO: 15, SEQ ID NO: 17 or SEQ ID NO: 19.
8. The method of any of embodiments 1 to 3 wherein the mogroside is Mog **IIIE** and has a structure of formula (V) 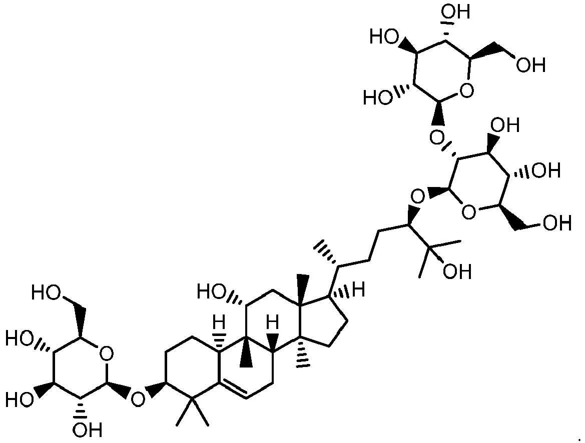
9. The method of embodiment 8 wherein where the method requires contacting mogroside IIIE the polypeptide having UDP-glycotransferase activity comprises an amino acid sequence having at least 40%, 45%, 50%, 55%, 60%, 65%, 70%, 75%, 80%, 85%, 90%, 95%, 96%, 97%, 98% or 99% sequence identity to any one of the following:, SEQ ID NO: 1, SEQ ID NO: 21 or SEQ ID NO: 23.
10. The method of any of the previous embodiments, wherein the cell is a yeast cell.
11. An expression vector comprising a nucleic acid molecule of nucleic acid encoding a UDP-glycotransferase activity, wherein the polypeptide comprises an amino acid sequence having at least 40%, 45%, 50%, 55%, 60%, 65%, 70%, 75%, 80%, 85%, 90%, 95%, 96%, 97%, 98% or 99% sequence identity to any one of the following: SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21 or SEQ ID NO: 23.
12. The vector of embodiment 11, wherein the vector is a prokaryotic vector, viral vector or a eukaryotic vector.
13. A host cell or a non-human host organism comprising
   (i) a nucleic acid molecule of nucleic acid encoding a UDP-glycotransferase activity, wherein the polypeptide comprises an amino acid sequence having at least 40%, 45%, 50%, 55%, 60%, 65%, 70%, 75%, 80%, 85%, 90%, 95%, 96%, 97%, 98% or 99% sequence identity to SEQ ID NO: 1, SEQ ID NO: 3, SEQ ID NO: 5, SEQ ID NO: 7, or SEQ ID NO: 9, SEQ ID NO: 11, SEQ ID NO: 13, SEQ ID NO: 15, SEQ ID NO: 17, SEQ ID NO: 19, SEQ ID NO: 21 or SEQ ID NO: 23.
   (ii) the vector of any one of embodiments 11 or 12.
14. The method of any of the previous embodiments wherein the siamenoside I obtained from step (i) is further formulated into a consumer product.
15. A recombinant cell comprising: siamenoside I having a structure of formula (I) and a gene encoding a polypeptide having UDP-glycotransferase activity.
16. A method for preparing siamenoside I having a structure of formula (I) comprising the steps of culturing a recombinant cell of embodiment 15.
17. A method of producing siamenoside I having the structure of formula (I): 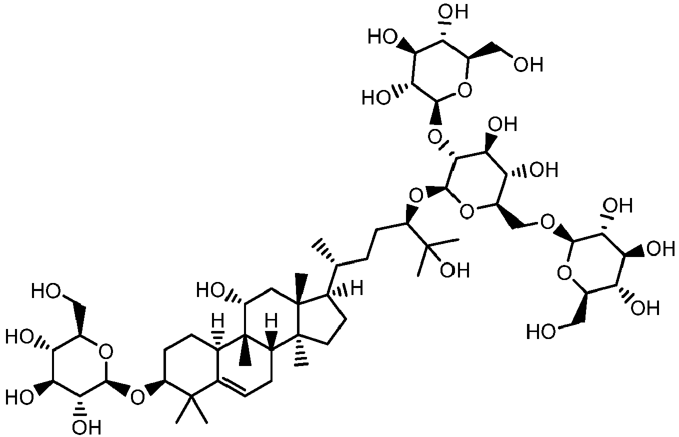 the method comprises contacting mogroside V having a structure of formula (VI) 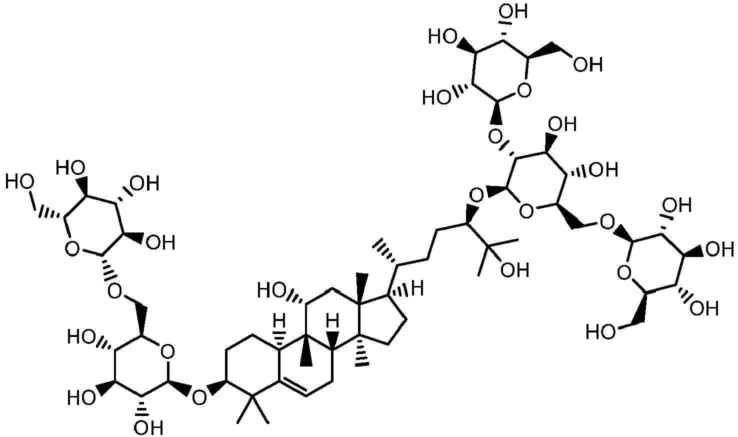 with a polypeptide having glucosidase activity.
18. The method of embodiment 17 wherein the polypeptide having glucosidase activity comprises an amino acid sequence having at least 40%, 45%, 50%, 55%, 60%, 65%, 70%, 75%, 80%, 85%, 90%, 95%, 96%, 97%, 98% or 99% sequence identity SEQ ID NO: 25.

## Claims

1. A method of producing siamenoside I having the structure of formula (I): 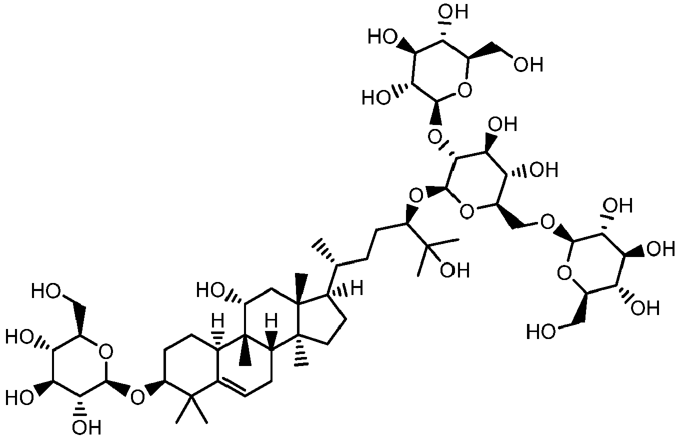 the method comprises contacting mogroside V having a structure of formula (VI) 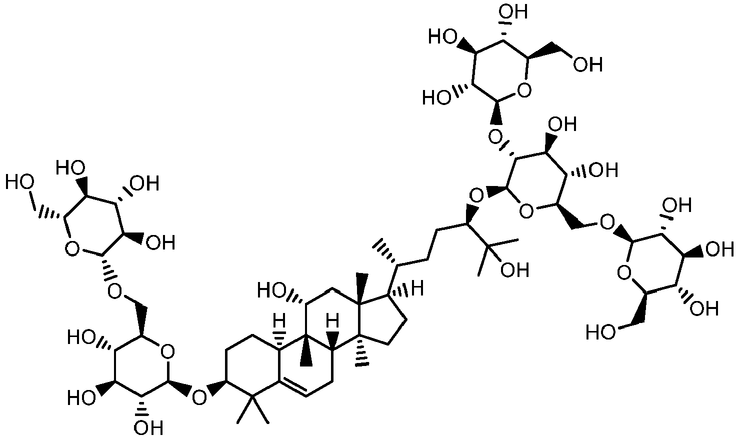 with a recombinant host cell expressing a polypeptide having glucosidase activity comprising an amino acid sequence having at least 80% sequence identity to SEQ ID NO: 25; optionally wherein the polypeptide having glucosidase activity comprises 90% sequence identity to SEQ ID NO: 25; further optionally wherein the polypeptide having glucosidase activity comprises 95% sequence identity to SEQ ID NO: 25; further optionally wherein the polypeptide having glucosidase activity comprises 99% sequence identity to SEQ ID NO: 25; further optionally wherein the polypeptide having glucosidase activity comprises SEQ ID NO: 25.

2. The method of claim 1, comprising cultivating the recombinant host cell in a culture medium under conditions in which the polypeptide having glucosidase activity is expressed.

3. The method of any one of claims 1-2, wherein one or more of the genes encoding a polypeptide having glucosidase activity is operably linked to a heterologous promoter.

4. The method of claim 3, wherein the heterologous promoter is a CMV, EF1a, SV40, PGK1, human beta actin, CAG, GAL1, GAL10, TEF1, GDS, ADH1, CaMV35S, Ubi, T7, T7lac, Sp6, araBAD, trp, lac, Ptac, pL promoter, or a combination thereof.

5. The method of any one of claims 1-4, wherein the recombinant host cell is a yeast cell.

6. The method of claim 5, wherein the yeast is selected from the group consisting of Candida, Sacccharaomyces, Saccharomycotina, Taphrinomycotina, Schizosaccharomycetes, Komagataella, Basidiomycota, Agaricomycotina, Tremellomycetes, Pucciniomycotina, Aureobasidium, Coniochaeta, Rhodosporidium, and Microboryomycetes.

7. The method of any one of claims 1-4, wherein the recombinant host cell is a *Saccharomyces cerevisiae* cell or a *Yarrowia lipolytica* cell.

8. The method of any one of claims 1-7, further comprising isolating siamenoside I.

9. The method of claim 8, wherein isolating siamenoside I comprises lysing the recombinant host cell.

10. The method of claim 8, wherein isolating siamenoside I comprises isolating siamenoside I from the culture medium.

11. The method of any one of claims 1-10, wherein the recombinant host cell is fed a mogrol precursor or mogroside precursor during cell growth or after cell growth.

12. The method of any one of claims 1-11, comprising contacting the recombinant host cell with monk fruit (*Siraitia grosvenorii)* extract.

13. The method of any one of claims 1-2, wherein the contacting takes place in the absence of glucose supplementation.

14. A recombinant cell comprising siamenoside I having the structure of formula (I): 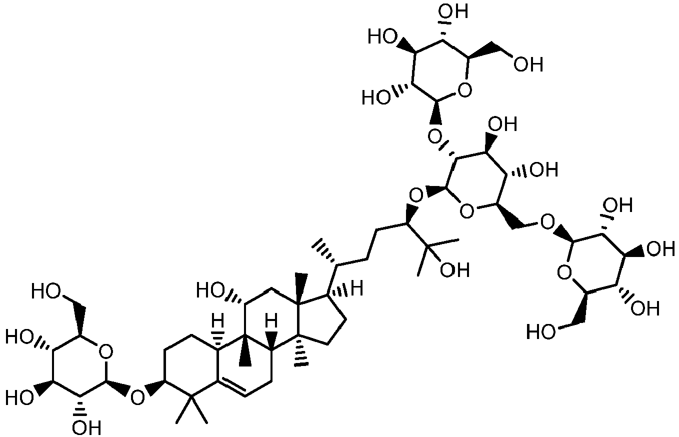 and a gene encoding a polypeptide having glucosidase activity, wherein the gene is a heterologous gene to the recombinant cell.
